(19) Europäisches Patentamt
European Patent Office
Office européen des brevets

(11) **EP 4 574 043 A1**

(12) **EUROPEAN PATENT APPLICATION**
published in accordance with Art. 153(4) EPC

(43) Date of publication:
**25.06.2025  Bulletin 2025/26**

(21) Application number: 23888868.9

(22) Date of filing: **09.08.2023**

(51) International Patent Classification (IPC):
*A61B 5/276* (2021.01)    *A61B 5/00* (2006.01)
*G06F 1/16* (2006.01)    *G06F 1/3231* (2019.01)
*G06F 11/30* (2006.01)    *G06F 11/32* (2006.01)

(52) Cooperative Patent Classification (CPC):
A61B 5/00; A61B 5/276; G06F 1/16; G06F 1/3231;
G06F 11/30; G06F 11/32

(86) International application number:
**PCT/KR2023/011774**

(87) International publication number:
**WO 2024/101601 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 07.11.2022  KR 20220147132
25.11.2022  KR 20220160031

(71) Applicant: Samsung Electronics Co., Ltd.
Suwon-si, Gyeonggi-do 16677 (KR)

(72) Inventors:
• **PARK, Jaehyuck**
  **Suwon-si, Gyeonggi-do 16677 (KR)**
• **JEONG, Daeung**
  **Suwon-si, Gyeonggi-do 16677 (KR)**
• **CHANG, Namseok**
  **Suwon-si, Gyeonggi-do 16677 (KR)**

(74) Representative: **Appleyard Lees IP LLP**
**15 Clare Road**
**Halifax HX1 2HY (GB)**

(54) **WEARABLE ELECTRONIC DEVICE AND FUNCTION CONTROL METHOD USING SAME**

(57)    This wearable electronic device may include: a housing including a first surface, a second surface opposite to the first surface, and a side surface surrounding the first surface and the second surface; a first electrode and a second electrode arranged on the second surface; a third electrode and a fourth electrode arranged on the side surface; and a processor operatively connected to the first electrode, the second electrode, the third electrode, and the fourth electrode. The processor may: repeatedly identify a parasitic impedance value of at least one of the first electrode, the second electrode, the third electrode, and the fourth electrode at designated time points; if the parasitic impedance value of the at least one electrode exceeds a designated first value, identify a resistance value of at least one of the third electrode and the fourth electrode; if the resistance value of at least one of the third electrode and the fourth electrode is less than or equal to a designated second value, identify whether at least one of the third electrode and the fourth electrode is short-circuited; and if a short circuit has occurred in at least one of the third electrode and the fourth electrode due to a conductor, deactivate some functions of the wearable electronic device.

EP 4 574 043 A1

# FIG. 6

600

START

↓

REPEATEDLY IDENTIFY PARASITIC IMPEDANCE VALUE OF AT LEAST ONE ELECTRODE AT SPECIFIED TIME POINTS — 610

↓

IF PARASITIC IMPEDANCE VALUE EXCEEDS FIRST SPECIFIED VALUE, IDENTIFY RESISTANCE VALUE OF THIRD ELECTRODE AND/OR FOURTH ELECTRODE — 620

↓

IF RESISTANCE VALUE OF THIRD ELECTRODE AND/OR FOURTH ELECTRODE IS LESS THAN OR EQUAL TO SECOND SPECIFIED VALUE, DETERMINE WHETHER THIRD ELECTRODE AND/OR FOURTH ELECTRODE ARE SHORTED — 630

↓

IF A SHORT OCCURS DUE TO A CONDUCTOR AT THIRD ELECTRODE AND/OR FOURTH ELECTRODE, DEACTIVATE SOME FUNCTIONS OF WEARABLE ELECTRONIC DEVICE — 640

↓

END

**Description**

[Technical Field]

An embodiment of the disclosure relates to a wearable electronic device and a method for controlling functions using the same.

[Background Art]

With the advancement of digital technology, various types of electronic devices such as mobile communication terminals, personal digital assistants (PDAs), electronic notebooks, smartphones, tablet personal computers (PCs), and wearable electronic devices are being widely used. In these electronic devices, to support and increase their functionality, hardware and/or software aspects of electronic devices are continuously being improved.

For example, a wearable electronic device may include electrodes for measuring biometric signals, and may use the electrodes to obtain biometric signals, such as an electrocardiogram (ECG), an electroencephalogram (EEG), and/or an electromyography (EMG). Since biometric signals can be measured inaccurately when affected by environmental factors such as temperature, humidity, and water, the user may deactivate the touch functionality of the wearable electronic device, for example, when engaging in a water-based activity (e.g., swimming).

The above information may be provided as background art for the purpose of assisting in understanding the disclosure. No claim or determination is made as to whether any of the above is applicable as prior art related to the disclosure.

[Disclosure of Invention]

[Technical Problem]

However, if the wearable electronic device is exposed to water or is submerged in water and the touch functionality is not deactivated by the user, unnecessary touching due to water may result in rapid battery discharge and corrosion may occur in the electrodes used to measure biometric signals.

The wearable electronic device according to an embodiment of the disclosure may identify a state in which the wearable electronic device is in contact with water or submerged in water, and deactivate the touch functionality, based on a parasitic impedance value of at least one electrode among a plurality of electrodes arranged on the rear and side surfaces of the wearable electronic device, a resistance value of the at least one electrode, and a contact impedance value of the at least one electrode.

[Solution to Problem]

A wearable electronic device according to an embodiment of the disclosure may include a housing that includes a first surface, a second surface opposite to the first surface, and a side surface surrounding the first surface and the second surface. In an embodiment, the wearable electronic device may include a first electrode and a second electrode disposed on the second surface of the housing. In an embodiment, the wearable electronic device may include a third electrode and a fourth electrode disposed on the side surface of the housing. In an embodiment, the wearable electronic device may include a processor operably connected to the first electrode, the second electrode, the third electrode, and the fourth electrode. In an embodiment, the processor may repeatedly identify the parasitic impedance value of at least one electrode among the first electrode, the second electrode, the third electrode, and the fourth electrode at specified time points. In an embodiment, the processor may identify the resistance value of at least one of the third electrode or the fourth electrode in case that the parasitic impedance value of the at least one electrode exceeds a first specified value. In an embodiment, the processor may determine whether at least one of the third electrode or the fourth electrode is shorted in case that the resistance value of at least one of the third electrode or the fourth electrode is less than or equal to a second specified value. In an embodiment, the processor may deactivate some functions of the wearable electronic device in case that a short has occurred due to a conductor in at least one of the third electrode or the fourth electrode.

A function control method of the wearable electronic device according to an embodiment of the disclosure may include repeatedly identifying the parasitic impedance value of at least one electrode among a first electrode, a second electrode, a third electrode, and a fourth electrode at specified time points. In an embodiment, the function control method of the wearable electronic device may include identifying the resistance value of at least one of the third electrode or the fourth electrode in case that the parasitic impedance value of the at least one electrode exceeds a first specified value. In an embodiment, the function control method of the wearable electronic device may include determining whether at least one of the third electrode or the fourth electrode is shorted in case that the resistance value of at least one of the third electrode or the fourth electrode is less than or equal to a second specified value. In an embodiment, the function control method of the wearable electronic device may include deactivating some functions of the wearable electronic device in case that a short has occurred due to a conductor in at least one of the third electrode or the fourth electrode.

According to an embodiment of the disclosure, a non-transitory computer-readable storage medium (or, computer program product) storing one or more programs may be described. The one or more programs according to an embodiment may include instructions that are configured to, when executed by a processor of an electronic device, repeatedly identify the parasitic impedance value of at least one electrode among a first electrode, a second electrode, a third electrode, and a fourth electrode at specified time points. The one or more programs may include instructions that are configured to, when executed by the processor of the electronic device, identify the resistance value of at least one of the third electrode or the fourth electrode in case that the parasitic impedance value of the at least one electrode exceeds a first specified value. The one or more programs may include instructions that are configured to, when executed by the processor of the electronic device, determine whether at least one of the third electrode or the fourth electrode is shorted in case that the resistance value of at least one of the third electrode or the fourth electrode is less than or equal to a second specified value. The one or more programs may include instructions that are configured to, when executed by the processor of the electronic device, deactivate some functions of the wearable electronic device in case that a short has occurred due to a conductor in at least one of the third electrode or the fourth electrode.

[Advantageous Effects of Invention]

The wearable electronic device according to an embodiment of the disclosure may identify the state where the wearable electronic device is in contact with water or submerged in water based on a parasitic impedance value, a resistance value, and a contact impedance value of at least one electrode among a plurality of electrodes arranged on the rear and side surfaces of the wearable electronic device and deactivate the touch functionality, preventing corrosion that may occur in the electrodes for measuring biometric signals. Further, the wearable electronic device may prevent battery discharge that may occur owing to unnecessary touching due to water by deactivating the touch functionality.

[Brief Description of Drawings]

FIG. 1 is a block diagram of an electronic device in a network environment according to an embodiment of the disclosure.

FIG. 2 is a front perspective view of a wearable electronic device according to an embodiment of the disclosure.

FIG. 3 is a rear perspective view of the wearable electronic device of FIG. 2 according to an embodiment of the disclosure.

FIG. 4 is an exploded perspective view of the wearable electronic device of FIG. 2 according to an embodiment of the disclosure.

FIG. 5 is a block diagram illustrating a wearable electronic device according to an embodiment of the disclosure.

FIG. 6 is a flowchart illustrating a function control method of the wearable electronic device according to an embodiment of the disclosure.

FIG. 7 is a diagram for depicting a method for identifying a parasitic impedance value of at least one electrode according to an embodiment of the disclosure.

FIG. 8 is a diagram illustrating a method for detecting moisture in the wearable electronic device based on the parasitic impedance value of at least one electrode according to an embodiment of the disclosure.

FIG. 9 is a diagram illustrating a method for detecting moisture in the wearable electronic device based on the resistance value of at least one electrode according to an embodiment of the disclosure.

FIG. 10A is a diagram illustrating electrical connections of a plurality of electrodes when biometric information is obtained in a state where the wearable electronic device is worn according to an embodiment of the disclosure.

FIG. 10B is a diagram illustrating electrical connections of a plurality of electrodes when biometric information is not obtained in a state where the wearable electronic device is worn according to an embodiment of the present disclosure.

FIG. 10C is a diagram illustrating a method for monitoring the impedance of at least one electrode by using resistance according to an embodiment of the disclosure.

FIG. 10D is a diagram illustrating a method for monitoring the impedance of at least one electrode by using a current according to one embodiment of the present disclosure.

FIG. 11 is a diagram illustrating a moisture detection circuit of the wearable electronic device according to an embodiment of the disclosure.

FIG. 12 is a flowchart illustrating the operation of FIG. 6 in detail according to an embodiment of the disclosure.

FIG. 13 is a flowchart illustrating a method for the wearable electronic device to output a moisture removal notification according to an embodiment of the disclosure.

FIG. 14 is a flowchart illustrating a function control method of the wearable electronic device according to an embodiment of the disclosure.

[Mode for the Invention]

Hereinafter, embodiments of the present disclosure will be described in detail with reference to the drawings so that those skilled in the art can readily carry out the disclosure. However, the disclosure may be implemented in various different forms and is not limited to those embodiments described herein. In connection with the description of the drawings, the same or similar reference symbols may be used for identical or similar components. Additionally, in the drawings and related descriptions, descriptions of well-known functions and configurations may be omitted for clarity and brevity.

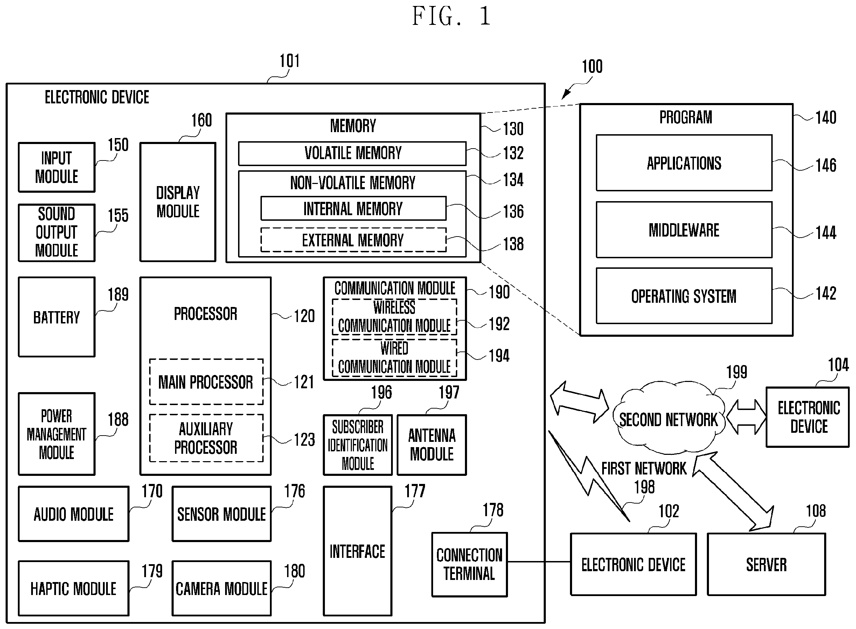

FIG. 1 is a block diagram illustrating an electronic device 101 in a network environment 100 according to various embodiments.

Referring to FIG. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connection terminal 178, a haptic module 179, a camera module 180, a power management module 188, a battery 189, a communication module 190, a subscriber identification module (SIM) 196, or an antenna module 197. In some embodiments, at least one of the components (e.g., the connection terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134. The non-volatile memory 134 may include an internal memory 136 and/or an external memory 138.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) (e.g., speaker or headphone) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., through wires) or wirelessly. According to an embodiment, the interface 177 may include, for example, a high-definition multimedia interface (HDMI), a universal serial bus (USB) interface, a secure digital (SD) card interface, or an audio interface.

The connection terminal 178 may include a connector via which the electronic device 101 may be physically connected with the external electronic device (e.g., the electronic device 102). According to an embodiment, the connection terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., an application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth™, Wi-Fi direct, or infrared data association (IrDA)) or the second network 199 (e.g., a long-range communication network, such as a legacy cellular network, a fifth generation (5G) network, a next-generation communication network, the Internet, or a computer network (e.g., LAN or wide area network (WAN))). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large-scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., an mmwave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

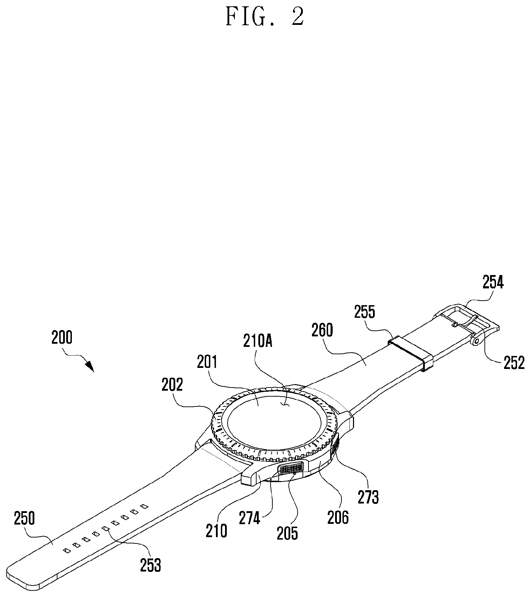

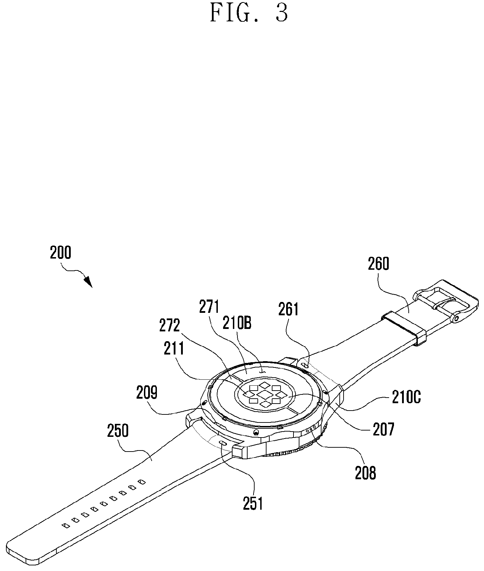

FIG. 2 is a front perspective view of a wearable electronic device 200 according to various embodiments. FIG. 3 is a rear perspective view of the wearable electronic device 200 of FIG. 2 according to various embodiments.

With reference to FIGS. 2 and 3, in one embodiment, the wearable electronic device 200 may include a housing 210 including a first surface (or, front surface) 210A, a second surface (or, rear surface) 210B, and a side surface 210C surrounding the space between the first surface 210A and the second surface 210B, and fastening members 250 and connected to at least a portion of the housing 210 and configured to detachably fasten the wearable electronic device 200 to a body part (e.g., wrist, ankle, etc.) of the user. In another embodiment (not shown), the housing 210 may refer to a structure forming some of the first surface 210A, the second surface 210B, and the side surface 210C in FIG. 2. In one embodiment, the first surface 210A may be formed by a front plate 201 that is substantially transparent at least in part (e.g., glass plate containing various coating layers, or polymer plate). The second surface 210B may be formed by a rear plate 207 that is substantially opaque. The rear plate 207 may be made of, for example, coated or colored glass, ceramic, polymer, metal (e.g., aluminum, stainless steel (STS), or magnesium), or a combination thereof. The side surface 210C is coupled to the front plate 201 and the rear plate 207 and may be formed by a side bezel structure (or, side member) 211 containing metal and/or polymer. In a certain embodiment, the rear plate 207 and the side bezel structure 211 may be integrally formed and contain the same material (e.g., metal material such as aluminum). The fastening members 250 and 260 may be made of various materials and formed in various shapes. For example, the fastening members 250 and 260 may be formed as a single body or as plural unit links that are movable with each other, by woven material, leather, rubber, urethane, metal, ceramic, or a combination thereof.

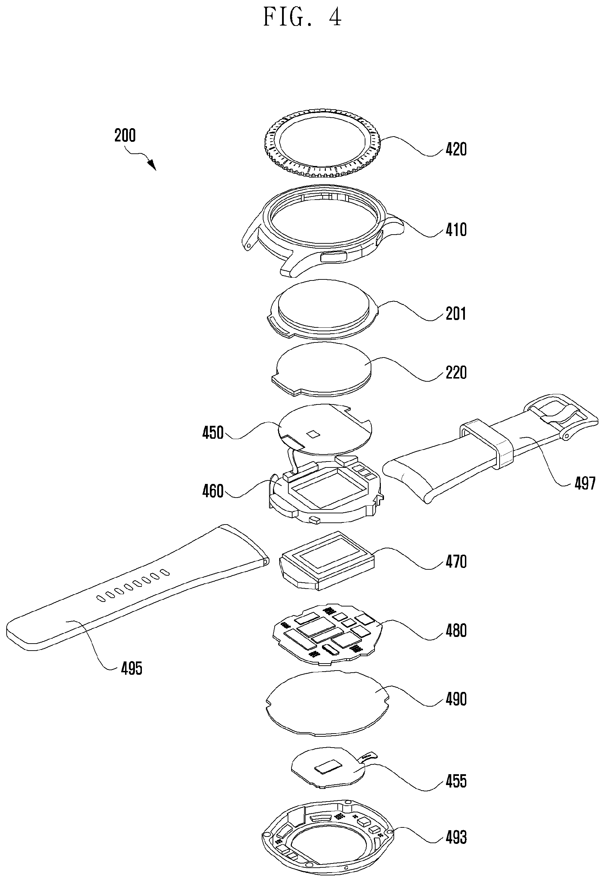

In one embodiment, the wearable electronic device 200 may include at least one of a display (e.g., display 220 in FIG. 4), an audio module 205 and 208, a sensor module 211, key input device 202, or a connector hole 209. In a certain embodiment, the wearable electronic device 200 may include at least one of the components (e.g., key input device 202, connector hole 209, or sensor module 211) may be removed from the wearable electronic device 200, or a different component may be added to the wearable electronic device 200.

The display 220 can be viewed through, for example, a significant portion of the front plate 201. The display 220 may have a shape corresponding to the shape of the front plate 201 and may have one of various shapes such as a circle, an ellipse, and a polygon. The display 220 may be disposed in combination with or adjacent to a touch sensing circuit, a pressure sensor capable of measuring the intensity (pressure) of a touch.

The audio module 205 and 208 may include a microphone hole 205 and a speaker hole 208. In the microphone hole 205, a microphone for picking up external sounds may be disposed therein, and plural microphones may be arranged to sense the direction of sound in a certain embodiment. The speaker hole 208 can be used for an external speaker and a call receiver. In a certain embodiment, the speaker hole 208 and the microphone hole 205 may be implemented as a single hole, or a speaker (e.g., piezo speaker) may be included without the speaker hole 208.

The sensor module 211 may generate an electrical signal or data value corresponding to an internal operating state of the wearable electronic device 200 or an external environmental state.

In an embodiment, the sensor module 211 may include a plurality of electrodes for measuring biometric signals. For example, the plurality of electrodes including a first electrode 271, a second electrode 272, a third electrode 273, and/or a fourth electrode 274.

In an embodiment, the plurality of electrodes may be arranged so as to come into contact with the user's body. For example, the first electrode 271 and the second electrode 272 may be disposed on the second surface (or, rear surface) 210B of the wearable electronic device 200. As the first electrode 271 and the second electrode 272 are disposed on the second surface (or, rear surface) 210B of the wearable electronic device 200, when the wearable electronic device 200 is worn (or, fixed) on a portion of the user's body (e.g., wrist), they may come into contact with the portion of the user's body (e.g., wrist). In an embodiment, the third electrode 273 and the fourth electrode 274 may be disposed on the side surface 210C of the wearable electronic device 200. As the third electrode 273 and the fourth electrode 274 are disposed on the side surface 210C of the wearable electronic device 200, they may come into contact with a user's finger (e.g., the finger of the hand that is not wearing the wearable electronic device 200). In an embodiment, the electrodes are described as being disposed on the rear surface 210B and the side surface 210C of the wearable electronic device 200, but without being limited thereto.

In an embodiment, the plurality of electrodes may be electrically isolated from each other.

In an embodiment, the sensor module 211 may obtain an electrical signal from a portion of the user's body by using the first electrode 271, the second electrode 272, the third electrode 273, and/or the fourth electrode 274 of the wearable electronic device 200, and obtain biometric information of the user based on the obtained electrical signal.

In an embodiment, the sensor module 211 can detect various biometric information of the user (e.g., photoplethysmography (PPG), electrocardiogram (ECG), galvanic skin response (GSR), electroencephalogram (EEG), and/or bioelectrical impedance analysis (BIA)) by using the plurality of electrodes.

The wearable electronic device 200 may further include a sensor module (not shown) including at least one of, for example, a gesture sensor, a gyro sensor, an air pressure sensor, a magnetic sensor, an acceleration sensor, a grip sensor, a color sensor, an infrared (IR) sensor, a temperature sensor, a humidity sensor, or an illuminance sensor.

The key input device 202 may include a wheel key 202 disposed on the first surface 210A of the housing 210 and rotatable in at least one direction. The wheel key 202 may have a shape corresponding to the shape of the front plate 201. In another embodiment, the key input device 202 may be implemented in other forms, such as soft keys, on the display 220.

The connector hole 209 may accommodate a connector (e.g., USB connector) for transmitting and receiving power and/or data to and from an external electronic device, and may include another connector hole (not shown) that can accommodate a connector for transmitting and receiving an audio signal to and from an external electronic device. The wearable electronic device 200 may further include, for example, a connector cover (not shown) that covers at least a portion of the connector hole 209 and blocks foreign substances from entering the connector hole 209.

The fastening members 250 and 260 may be detachably fastened to at least a portion of the housing 210 by using locking members 251 and 261. The fastening members 250 and 260 may include one or more of a fixing member 252, fixing member fastening holes 253, a band guide member 254, and a band fixing ring 255.

The fixing member 252 may be configured to fix the housing 210 and the fastening members 250 and 260 to a body part (e.g., wrist, or ankle) of the user. The fixing member fastening holes 253 may fix the housing 210 and the fastening members 250 and 260 to a body part of the user in correspondence to the fixing member 252. The band guide member 254 may be configured to limit the range of movement of the fixing member 252 when the fixing member 252 engages with a fixing member fastening hole 253, so that the fastening members 250 and 260 may be fastened in close contact to a body part of the user. The band fixing ring 255 may limit the range of movement of the fastening members 250 and 260 while the fixing member 252 and the fixing member fastening hole 253 are fastened.

FIG. 4 is an exploded perspective view of a wearable electronic device according to various embodiments.

With reference to FIG. 4, the wearable electronic device 200 may include a hosing 410 (e.g., housing 210 in FIG. 2), a wheel key 420 (e.g., wheel key 202 in FIG. 2), a front plate 201, a display 220, a first antenna 450, a second antenna 455, a support member 460 (e.g., bracket), a battery 470, a printed circuit board 480, a sealing member 490, a rear plate 493, and/or fastening members 495, 497 (e.g., fastening members 250 and 260 in FIGS. 2 and 3). At least one of the components of the wearable electronic device 200 may be identical or similar to at least one of the components of the wearable electronic device 200 of FIGS. 2 or 3, and repeated descriptions are omitted herein.

The support member 460 disposed inside the wearable electronic device 200 may be formed to be connected to the hosing 410 or be integrally formed with the hosing 410. The support member 460 may be made of, for example, a metal material and/or a non-metal (e.g., polymer) material. The support member 460 may have one surface coupled to the display 220, or the other surface coupled to the printed circuit board 480. A processor (e.g., processor 120 in FIG. 1), a memory (e.g., memory 130 in FIG. 1), and/or an interface (e.g., interface 177 in FIG. 1) may be mounted on the printed circuit board 480.

The battery 470 (e.g., battery 189 in FIG. 1) is a device for supplying power to at least one component of the wearable electronic device 200, and may include, for example, a non-rechargeable primary cell, a rechargeable secondary cell, or a fuel cell. At least a portion of the battery 470 may be disposed substantially on the same plane as, for example, the printed circuit board 480. The battery 470 may be disposed as a single body within the wearable electronic device 200 or may be detachably disposed from the wearable electronic device 200.

The first antenna 450 may be disposed between the display 220 and the support member 241. The first antenna 450 may include, for example, a near field communication (NFC) antenna, a wireless charging antenna, and/or a magnetic secure transmission (MST) antenna. For example, the antenna 243 may perform short-range communication with an external device, wirelessly transmit or receive power required for charging, and transmit a short-range communication signal or a magnetic-based signal including payment data. In another embodiment, an antenna structure may be formed by using portions of the housing 410 and/or the support member 460 or a combination thereof.

The second antenna 455 may be disposed between the printed circuit board 480 and the rear plate 493. The second antenna 455 may include, for example, a near field communication (NFC) antenna, a wireless charging antenna, and/or a magnetic secure transmission (MST) antenna. For example, second antenna 455 may perform short-range communication with an external device, wirelessly transmit or receive power required for charging, and transmit a short-range communication signal or a magnetic-based signal including payment data. In another embodiment, an antenna structure may be formed by using portions of the housing 410 and/or the rear plate 493 or a combination thereof.

The sealing member 490 may be positioned between the hosing 410 and the rear plate 493. The sealing member 490 may be configured to block moisture and foreign matter flowing into the space surrounded by the hosing 410 and the rear plate 493 from the outside.

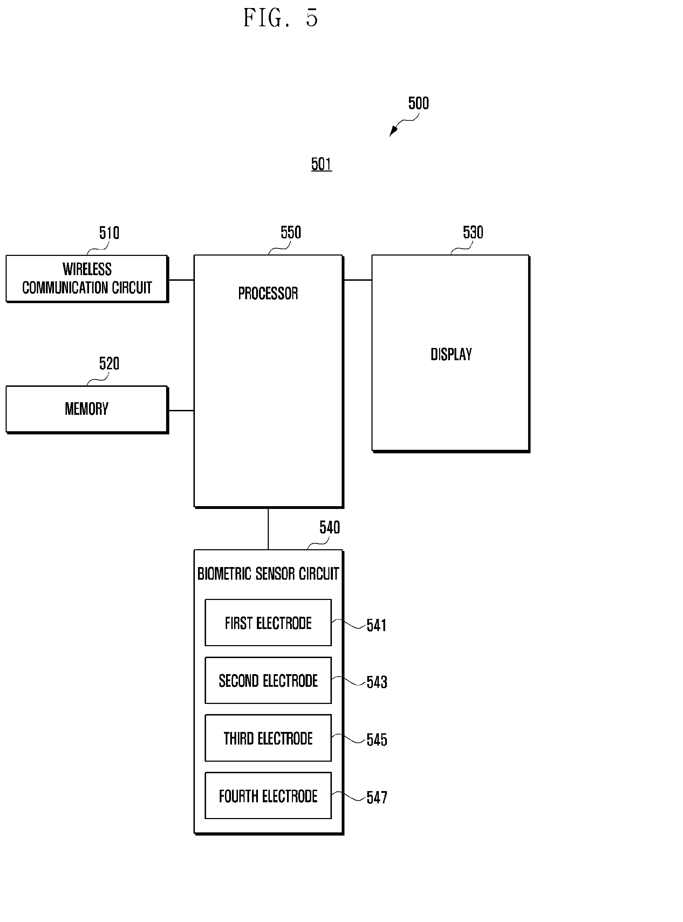

FIG. 5 is a block diagram 500 illustrating a wearable electronic device 501 according to an embodiment of the disclosure.

With reference to FIG. 5, the wearable electronic device 501 (e.g., electronic device 101 in FIG. 1, wearable electronic device 200 of FIGS. 2 to 4) may include a wireless communication circuit 510 (e.g., communication module 190 in FIG. 1), a memory 520 (e.g., memory 130 in FIG. 1), a display 530 (e.g., display module 160 in FIG. 1, display 220 in FIG. 4), a biometric sensor circuit 540 (e.g., sensor module 176 in FIG. 1, sensor module 211 in FIG. 2), and/or a processor 550 (e.g., processor 120 in FIG. 1).

According to various embodiments, the wireless communication circuit 510 (e.g., communication module 190 in FIG. 1) may establish a communication channel with an external electronic device (e.g., electronic device 102 in FIG. 1) and support transmitting and receiving various data with the external electronic device.

According to various embodiments, the memory 520 (e.g., memory 130 in FIG. 1) may store programs for processing and controlling the processor 550 (e.g., programs 140 in FIG. 1), an operating system (OS) (e.g., operating system 142 in FIG. 1), various applications, and/or input/output data, and may store a program for controlling the overall operation of the wearable electronic device 501. The memory 520 may store various instructions that can be executed by the processor 550.

In an embodiment, the memory 520 may store instructions for repeatedly identifying the parasitic impedance value of at least one of the first electrode 541, the second electrode 543, the third electrode 545, and the fourth electrode 547 at designated time points under the control of the processor 550. The memory 520 may store instructions for identifying whether moisture is detected in the wearable electronic device 501 by identifying the parasitic impedance value of at least one electrode, the resistance value of at least one electrode, and the short of at least one electrode under the control of the processor 550. The memory 520 may store instructions for deactivating some functions of the wearable electronic device 501 under the control of the processor 550 upon determining that moisture is detected in the wearable electronic device 501. The memory 520 may store instructions for determining whether moisture is detected in at least one electrode based on the parasitic impedance value of the at least one electrode under the control of the processor 550. The memory 520 may store instructions for outputting a moisture removal notification under the control of the processor 550 upon determining that moisture is detected at at least one electrode.

According to various embodiments, the display 530 (e.g., display module 160 in FIG. 1, display 220 in FIG. 4) may be implemented with, but not limited to, a liquid crystal display (LCD), a light-emitting diode (LED) display, a micro LED display, a quantum dot (QD) display, or an organic light-emitting diode (OLED) display. In an embodiment, the display 530 may be formed as a touchscreen that detects a touch input and/or proximity (or, hovering) input using a portion of the user's body (e.g., finger) or an input gadget (e.g., stylus pen).

In an embodiment, the display 530 may display a user interface related to measuring a biometric signal under the control of the processor 550. The display 530 may display a user interface related to biometric information obtained through the biometric sensor circuit 540 under the control of the processor 550. The display 530 may, under the control of the processor 550, display a user interface including a moisture removal notification when moisture is detected in the wearable electronic device 501.

According to various embodiments, the biometric sensor circuit 540 (e.g., sensor module 176 in FIG. 1, sensor module 211 in FIG. 2) may sense at least one biometric signal.

In an embodiment, the biometric sensor circuit 540 may include a first electrode 541 (e.g., first electrode 271 in FIG. 3), a second electrode 543 (e.g., second electrode 272 in FIG. 3), a third electrode 545 (e.g., third electrode 273 in FIG. 2), and/or a fourth electrode 547 (e.g., fourth electrode 274 in FIG. 2).

In an embodiment, the biometric sensor circuit 540 is described as including four electrodes, but it is not limited thereto. For example, the biometric sensor circuit 540 may include more than four electrodes.

In an embodiment, contact impedance may be caused when an electrode and the user's body come into contact, and the biometric sensor circuit 540 may measure the contact impedance caused by the electrode. In an embodiment, the biometric sensor circuit 540 may measure body impedance by receiving at least some of the reflected light that is reflected from the light output to the user's body.

In an embodiment, the electrodes of the biometric sensor circuit 540 may be in contact with different body portions. For example, the first electrode 541 and the second electrode 543 may be disposed on the second surface (or, rear surface) of the wearable electronic device 501 (e.g., second surface 210B in FIG. 3) so that they can come into contact with a portion of the user's body (e.g., wrist) when the wearable electronic device 501 is worn (or, fixed) on the portion of the user's body. The third electrode 545 and the fourth electrode 547 may be disposed on the side surface (e.g., side surface 210C in FIG. 3) of the wearable electronic device 501 so that they can come into contact with a user's finger (e.g., a finger of the hand not wearing the wearable electronic device 501).

In an embodiment, the biometric sensor circuit 540 may measure a first parasitic impedance value generated when the first electrode 541 contacts the wrist, a second parasitic impedance value generated when the second electrode 543 contacts the wrist, a third parasitic impedance value generated when the third electrode 545 contacts a finger, and/or a fourth parasitic impedance value generated when the fourth electrode 547 contacts a finger, and transfer the same to the processor 550.

In an embodiment, the biometric sensor circuit 540 may measure the resistance value of the third electrode 545 and/or the fourth electrode 547 and transfer it to the processor 550.

According to various embodiments, the processor 550 may include, for example, a micro controller unit (MCU), and may control a plurality of hardware components connected to the processor 550 by running an operating system (OS) or an embedded software program. The processor 550 may control a plurality of hardware components according to, for example, instructions (e.g., programs 140 in FIG. 1) stored in the memory 520.

In an embodiment, the processor 550 may repeatedly identify the parasitic impedance value of at least one electrode among the first electrode 541, the second electrode 543, the third electrode 545, and the fourth electrode 547 at specified time points. If the parasitic impedance value of the at least one electrode exceeds a specified first value, the processor 550 may determine that moisture is detected in the wearable electronic device 501 and may identify the resistance value of the third electrode 545 and/or the fourth electrode 547. If the resistance value of the third electrode 545 and/or the fourth electrode 547 is lower than or equal to a specified second value, the processor 550 may determine whether the third electrode 545 and/or the fourth electrode 547 is shorted. For example, if the resistance value of the third electrode 545 and/or the fourth electrode 547 is less than or equal to the specified second value, the processor 550 may determine that moisture is detected in the wearable electronic device 501. If a short occurs due to a conductor of the third electrode 545 and/or the fourth electrode 547, the processor 550 may deactivate some functions of the wearable electronic device 501. For example, if a short occurs due to a conductor contacting the third electrode 545 and the fourth electrode 547, the processor 550 may deactivate some functions of the wearable electronic device 501. For example, some functions of the wearable electronic device 501 may include, but not limited to, a touch function.

For example, if the parasitic impedance value of at least one electrode exceeds the specified first value, the resistance value of the third electrode 545 and/or the fourth electrode 547 is less than or equal to the specified second value, and a short occurs due to a conductor in the third electrode 545 and/or the fourth electrode 547, the processor 550 may determine that moisture is detected in the wearable electronic device 501 and deactivate some functions (e.g., touch function) of the wearable electronic device 501.

In an embodiment, the processor 550 may repeatedly identify the parasitic impedance value of at least one electrode at specified time points in a state where some functions (e.g., touch function) of the wearable electronic device 501 are deactivated. If the parasitic impedance value is less than or equal to the specified first value, the processor 550 may identify the resistance value of the third electrode 545 and/or the fourth electrode 547. If the resistance value of the third electrode 545 and/or the fourth electrode 547 exceeds the specified second value, the processor 550 may identify whether the third electrode 545 and/or the fourth electrode 547 is shorted. If no short occurs due to a conductor at the third electrode 545 and/or the fourth electrode 547, the processor 550 may activate those deactivated functions of the wearable electronic device 501. For example, if a short does not occur due to a conductor not contacting the third electrode 545 and the fourth electrode 547, the processor 550 may activate those deactivated functions (e.g., touch function) of the wearable electronic device 501. For example, if the parasitic impedance value of at least one electrode is less than or equal to the specified first value, the resistance value of the third electrode 545 and/or the fourth electrode 547 exceeds the specified second value, and a short does not occur due to a conductor at the third electrode 545 and/or the fourth electrode 547, the processor 550 may determine that moisture is not detected in the wearable electronic device 501 and activate those deactivated functions (e.g., touch function) of the wearable electronic device 501.

In an embodiment, if detecting removal of the wearable electronic device 501, the processor 550 may identify the parasitic impedance value of the first electrode 541 and/or the second electrode 543 to determine whether it exceeds a specified third value. If the parasitic impedance value of the first electrode 541 and/or the second electrode 543 exceeds the specified third value, the processor 550 may output a moisture removal notification as to the first electrode 541 and/or the second electrode 543. For example, the moisture removal notification may be output via the display 530, the audio module (e.g., audio module 170 in FIG. 1), and/or the haptic module (e.g., haptic module 179 in FIG. 1).

In one embodiment, if not detecting removal of the wearable electronic device 501, the processor 550 may detect an input for measuring a biometric signal through the third electrode 545 and/or the fourth electrode 547. The processor 550 may identify the parasitic impedance value of the third electrode 545 and/or the fourth electrode 547 to determine whether the parasitic impedance value of the third electrode 545 and/or the fourth electrode 547 exceeds a specified fourth value. If the parasitic impedance value of the third electrode 545 and/or the fourth electrode 547 exceeds the specified fourth value, the processor 550 may output a moisture removal notification as to the third electrode 545 and/or the fourth electrode 547. If the parasitic impedance value of the third electrode 545 and/or the fourth electrode 547 does not exceed the specified value, the processor 550 may measure a user's biometric signal by using the third electrode 545 and/or the fourth electrode 547.

The electronic device 101, 200 or 501 according to various embodiments may include a housing 210 that includes a first surface 210A, a second surface 210B opposite to the first surface 210A, and a side surface 210C surrounding the first surface 210A and the second surface 210B. In an embodiment, the electronic device 101, 200 or 501 may include a first electrode 271 or 541 and a second electrode 272 or 543 disposed on the second surface 210B of the housing 210. In an embodiment, the electronic device 101, 200 or 501 may include a third electrode 273 or 545 and a fourth electrode 274 or 547 disposed on the side surface 210C of the housing 210. In an embodiment, the electronic device 101, 200 or 501 may include a processor 550 operably connected to the first electrode 271 or 541, the second electrode 272 or 543, the third electrode 273 or 545, and the fourth electrode 274 or 547. In an embodiment, the processor 550 may repeatedly, at specified time points, identify the parasitic impedance value of at least one electrode among the first electrode 271 or 541, the second electrode 272 or 543, the third electrode 273 or 545, and the fourth electrode 274 or 547. In an embodiment, if the parasitic impedance value of the at least one electrode exceeds a specified first value, the processor 550 may identify the resistance value of at least one of the third electrode 273 or 545 or the fourth electrode 274 or 547. In an embodiment, if the resistance value of at least one of the third electrode 273 or 545 and the fourth electrode 274 or 547 is less than or equal to a specified second value, the processor 550 may determine whether at least one of the third electrode 273 or 545 or the fourth electrode 274 or 547 is shorted. In an embodiment, if a short has occurred due to a conductor in at least one of the third electrode 273 or 545 or the fourth electrode 274 or 547, the processor 550 may deactivate some functions of the wearable electronic device 101, 200 or 501.

In an embodiment, if a short has occurred due to a conductor in at least one of the third electrode 273 or 545 or the fourth electrode 274 or 547, the processor 550 may determine that moisture is detected in the wearable electronic device 101, 200 or 501.

In an embodiment, the first electrode 271 or 541 and the third electrode 273 or 545 may be connected to an AC current source. In an embodiment, the second electrode 272 or 543 and the fourth electrode 274 or 547 may be connected to a voltage detector.

In an embodiment, the processor 550 may perform calculations based on the AC current generated from the AC current source and the voltage signal generated from the voltage sensor to determine a parasitic impedance value according to parasitic components generated from the at least one electrode.

In an embodiment, the processor 550 may monitor the impedance between the third electrode 273 or 545 and the fourth electrode 274 or 547 to determine whether a conductor is in contact with at least one of the third electrode 273 or 545 or the fourth electrode 274 or 547.

In an embodiment, the processor 550 may utilize the resistance or current of at least one of the third electrode 273 or 545 or the fourth electrode 274 or 547 to determine whether a conductor is in contact with at least one of the third electrode 273 or 545 or the fourth electrode 274 or 547.

In an embodiment, if a short occurs due to a conductor coming into contact with at least one of the third electrode 273 or 545 or the fourth electrode 274 or 547 while applying resistance to or applying a current through at least one of the third electrode 273 or 545 or the fourth electrode 274 or 547, the processor 550 may deactivate some functions of the wearable electronic device 101, 200 or 501.

In an embodiment, the processor 550 may determine whether the wearable electronic device 101, 200 or 501 is removed. In an embodiment, upon detecting the removal of the wearable electronic device 101, 200 or 501, the processor 550 may identify the parasitic impedance value of at least one of the first electrode 271 or 541 or the second electrode 272 or 543. In an embodiment, if the parasitic impedance value of at least one of the first electrode 271 or 541 or the second electrode 272 or 543 exceeds a specified third value, the processor 550 may output a moisture removal notification as to at least one of the first electrode 271 or 541 or the second electrode 272 or 543.

In an embodiment, upon not detecting the removal of the wearable electronic device 101, 200 or 501, the processor 550 may determine whether an input for measuring a biometric signal through at least one of the third electrode 273 or 545 or the fourth electrode 274 or 547 is detected. In an embodiment, if an input for measuring a biometric signal through at least one of the third electrode 273 or 545 or the fourth electrode 274 or 547 is detected, the processor 550 may identify the parasitic impedance value of at least one of the third electrode 273 or 545 or the fourth electrode 274 or 547. In an embodiment, if the parasitic impedance value of at least one of the third electrode 273 or 545 or the fourth electrode 274 or 547 exceeds a specified fourth value, the processor 550 may output a moisture removal notification as to at least one of the third electrode 273 or 545 or the fourth electrode 274 or 547.

The wearable electronic device 101, 200 or 501 according to an embodiment may include a display 530. The wearable electronic device 101, 200 or 501 according to an embodiment may include an audio output circuit 170. The wearable electronic device 101, 200 or 501 according to an embodiment may include a haptic module 179. In an embodiment, the processor 550 may output a moisture removal notification as to at least one electrode through at least one of the display 530, the audio output circuit 170, or the haptic module 179.

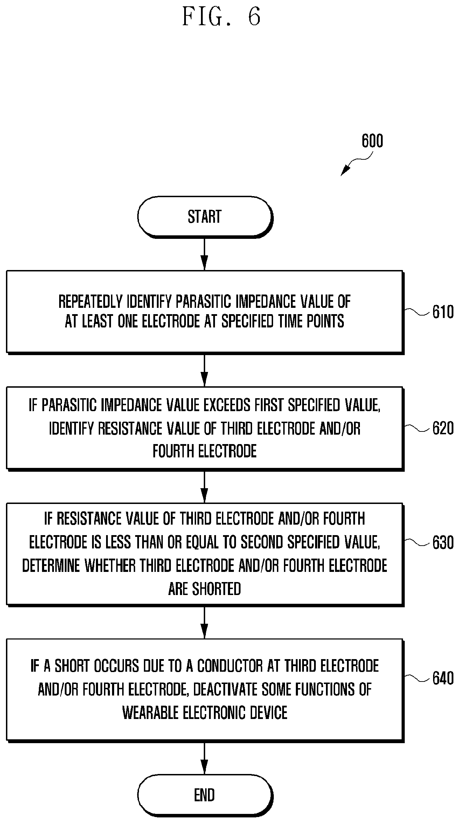

FIG. 6 is a flowchart 600 for describing a function control method of the wearable electronic device 501 according to an embodiment of the disclosure.

In the following embodiments, operations may be performed in sequence, but are not necessarily performed sequentially. For example, the order of operations may be changed, and at least two operations may be performed in parallel.

According to an embodiment, operations 610 to 640 may be understood to be executed by the processor (e.g., processor 550 in FIG. 5) of the wearable electronic device (e.g., wearable electronic device 501 of FIG. 5).

With reference to FIG. 6, at operation 610, the processor 550 of the wearable electronic device 501 may repeatedly identify the parasitic impedance value of at least one electrode at specified time points.

In an embodiment, the biometric sensor circuit (e.g., biometric sensor circuit 540 in FIG. 5) may include a bioelectrical impedance analysis (BIA) sensor. In an embodiment, the BIA sensor may include a plurality of electrodes. For example, the plurality of electrodes may include a first electrode (e.g., first electrode 541 in FIG. 5), a second electrode (e.g., second electrode 543 of FIG. 5), a third electrode (e.g., third electrode 545 in FIG. 5), and/or a fourth electrode (e.g., fourth electrode 547 in FIG. 5). For example, the first electrode 541 and the second electrode 543 may be disposed on the rear surface (e.g., rear surface 210B in FIG. 3) of the wearable electronic device 501 so that they can come into contact with the user's wrist when the wearable electronic device 501 is worn. The third electrode 545 and the fourth electrode 547 may be disposed on the side surface (e.g., side surface 210C in FIG. 3) of the wearable electronic device 501 so that they can come into contact with a user's finger (e.g., a finger of the hand not wearing the wearable electronic device 501).

In an embodiment, the processor 550 may repeatedly measure the impedance value of the user's body at specified time points by using the plurality of electrodes. For example, the periodicity of the specified time points may be, but not limited to, about 30 seconds.

In an embodiment, when the plurality of electrodes are in contact with the body, the plurality of electrodes and the user's body may form a closed circuit. For example, the first electrode 541 that comes into contact with the wrist (e.g., left wrist or right wrist) of the hand wearing the wearable electronic device 501 may be connected to an AC current source, and the second electrode 543 may be connected to a voltage detector. In addition, the third electrode 545 that comes into contact with a finger (e.g., right finger or left finger) of the hand not wearing the wearable electronic device 501 may be connected to the AC current source, and the fourth electrode 547 may be connected to the voltage detector.

In an embodiment, the parasitic impedance value may be obtained through calculations based on the AC current generated from the AC current source and the voltage signal of the voltage sensor connected to both electrodes (e.g., second electrode 543 and fourth electrode 547). In an embodiment, the parasitic impedance value may be obtained by computing the current of the AC current flowing out through the parasitic component generated at each electrode.

In an embodiment, the processor 550 may apply an AC current to the user's body, and calculate the impedance value (Z) of the electrode based on Equation 1 below. For example, the biometric sensor circuit 540 may apply a current of a specified AC current value (e.g., about 5 to 250 kHz) to the body connected to two electrodes (e.g., first electrode 541 and third electrode 545 connected to the AC current source). The biometric sensor circuit 540 may measure the voltage between two other electrodes (e.g., second electrode 543 and fourth electrode 547 connected to the voltage detector) to identify the impedance value of the body between the current loop formed between the portions where the electrodes are in contact.

[Equation 1]

$$Z = R + jX \text{ (R: resistance component, j: integer, X: reactance component)}$$

Equation 1 is an example to help understanding, and without being limited thereto, may be modified, applied, or expanded in various ways.

In an embodiment, at operation 620, if the parasitic impedance value exceeds a specified first value, the processor 550 may identify the resistance value of the third electrode 545 and/or the fourth electrode 547.

In an embodiment, determining whether the parasitic impedance value exceeds the stored first value may be an operation of determining whether moisture is detected in the wearable electronic device 501.

In an embodiment, the first electrode 541 and the second electrode 543 may be in contact with the wrist where current does not easily flow, and the third electrode 545 and the fourth electrode 547 may not allow current to easily flow as they are exposed to the air without contacting a user's finger. In this case, the parasitic impedance value of each electrode may be less than or equal to, but not limited to, about 100 pF.

In an embodiment, when moisture is detected as the wearable electronic device 501 is submerged immersed in water, as a state where electricity can flow relatively easily, the parasitic impedance value of the electrode in contact with the water may increase by about 1000 times.

In an embodiment, if it is identified that the parasitic impedance value of the electrode in contact with water increases by about 1000 times and exceeds the specified first value, the processor 550 may determine that moisture is detected in the wearable electronic device 501 and may identify the resistance value of the third electrode 545 and/or the fourth electrode 547.

In an embodiment, the first electrode 541 and the second electrode 543 that come into contact with the user's wrist may have low contact resistance, but the third electrode 545 and the fourth electrode 547 may not conduct current as they are exposed to the air without contacting the user's finger. In this case, the contact resistance of the third electrode 545 and the fourth electrode 547 may increase by about 50 times compared to when they come into contact with water.

In an embodiment, at operation 630, if the resistance value of the third electrode 545 and/or the fourth electrode 547 is less than or equal to the specified second value, the processor 550 may determine whether the third electrode 545 and/or the fourth electrode 547 are shorted.

As described above, if it is identified that the contact resistance of the third electrode 545 and the fourth electrode 547 does not increase by about 50 times and the resistance value of the third electrode 545 and/or the fourth electrode 547 is less than or equal to the specified second value, the processor 550 may determine that moisture is detected in the wearable electronic device 501 and perform a short-circuit identify of the third electrode 545 and/or the fourth electrode 547.

In an embodiment, the processor 550 may monitor the impedance between the electrodes to determine whether a conductor is in contact with one electrode (e.g., lead-off). For example, the processor 550 may monitor the impedance of the third electrode 545 and the fourth electrode 547 by using the resistance or current of the third electrode 545 and the fourth electrode 547 to identify whether a conductor is in contact with the third electrode 545 and the fourth electrode 547.

In an embodiment, at operation 640, if a short occurs due to a conductor at the third electrode 545 and/or the fourth electrode 547, the processor 550 may deactivate some functions of the wearable electronic device 501. For example, if a short occurs due to a conductor contacting the third electrode 545 and the fourth electrode 547, the processor 550 may deactivate some functions of the wearable electronic device 501. For example, those deactivated functions of the wearable electronic device 501 may include, but not limited to, the touch functionality.

In FIG. 6 according to various embodiments, if the parasitic impedance value of at least one electrode exceeds a specified first value, the resistance value of the third electrode 545 and/or the fourth electrode 547 is less than or equal to a specified second value, and a short occurs due to a conductor in the third electrode 545 and/or the fourth electrode 547, the processor 550 may determine that moisture is detected in the wearable electronic device 501 and may deactivate some functions (e.g., touch function) of the wearable electronic device 501. Upon determining that moisture is detected in the wearable electronic device 501, the processor 550 deactivates the touch functionality, thereby preventing battery discharge that may occur due to unnecessary touch caused by moisture, as well as preventing corrosion that may occur in the electrode due to moisture.

In FIG. 6 according to various embodiments, it has been described that if the parasitic impedance value of at least one electrode exceeds the specified first value, the resistance value of the third electrode 545 and/or the fourth electrode 547 is less than or equal to the specified second value, and a short occurs due to a conductor at the third electrode 545 and/or the fourth electrode 547, it is determined that moisture is detected in the wearable electronic device 501. But without being limited thereto, for example, the processor 550 may determine that moisture is detected in the wearable electronic device 501 based on at least two conditions (e.g., a condition in which the parasitic impedance value of at least one electrode exceeds a specified first value and a short occurs due to a conductor at the third electrode 545 and/or the fourth electrode 547 and/or a condition in which the resistance value of the third electrode 545 and/or the fourth electrode 547 is less than or equal to a specified second value and a short occurs due to a conductor at the third electrode 545 and/or the fourth electrode 547.

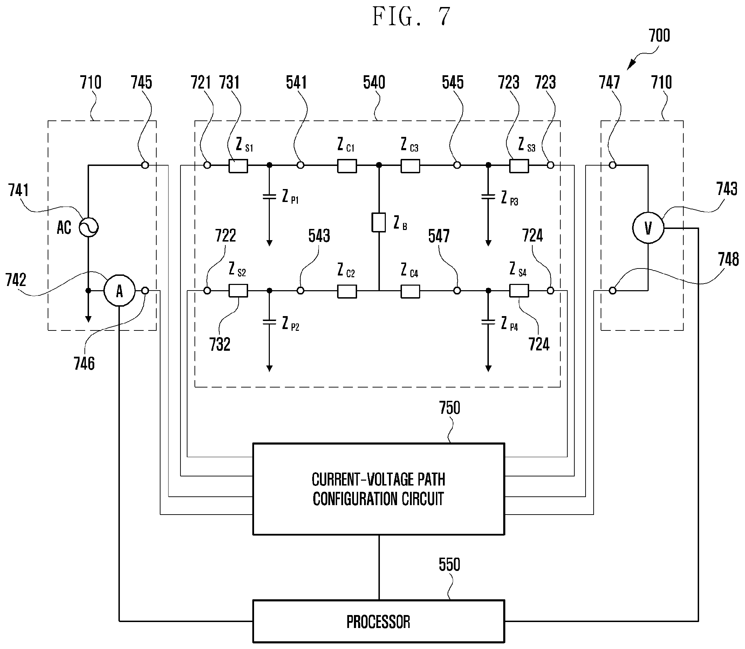

FIG. 7 is a diagram 700 for explaining a method for identifying the parasitic impedance value of at least one electrode according to an embodiment of the disclosure.

With reference to FIG. 7, the wearable electronic device 501 may include a biometric sensor circuit 540, a current-voltage measurement circuit 710, a current-voltage path configuration circuit 750, and/or a processor 550.

In an embodiment, the biometric sensor circuit 540 may include four electrodes 541, 543, 545 and 547, four ports 721, 722, 723 and 724, and/or four circuit elements 731, 732, 733 and 734. The electrodes 541, 543, 545 and 547 are disposed on the surface (e.g., rear surface 210B or side surface 201C in FIG. 3) of the wearable electronic device 501 so as to be in contact with the user's body, and thus may be electrically connected to each other through the body. The first electrode 541 may be electrically connected to the first port 721 via the first circuit element 731. The second electrode 543 may be electrically connected to the second port 722 via the second circuit element 732. The third electrode 545 may be electrically connected to the third port 723 via the third circuit element 733. The fourth electrode 547 may be electrically connected to the fourth port 724 via the fourth circuit element 734. Each of the circuit elements 731, 732, 733 and 734 may include electronic components (e.g., capacitor and resistor) to remove DC components from the electric signal flowing from the corresponding port to the corresponding electrode (or, flowing in opposite direction). The circuit elements 731, 732, 733 and 734 may respectively have $Z_{S1}$, $Z_{S2}$, $Z_{S3}$ and $Z_{S4}$ as impedance components given at the time of circuit design, and these impedance components may be named as characteristic impedance. Impedance components $Z_{C1}$, $Z_{C2}$, $Z_{C3}$ and $Z_{C4}$ may be generated respectively between the body and the electrodes 541, 543, 545 and 547, and these may be named as contact impedance. The contact impedance may vary depending on the surface condition (e.g., skin condition) of the body. The contact impedance may also vary depending on the frequency of the applied electrical signal. The impedance component $Z_B$ present between contact impedances is the component to be obtained, and $Z_B$ may be named as bio-impedance. Impedance components $Z_{P1}$, $Z_{P2}$, $Z_{P3}$ and $Z_{P4}$ that are not intended at the time of circuit design may be parasitic respectively between the electrodes 541, 543, 545 and 547 and the ground (e.g., ground of the wearable electronic device 501), and these may be named parasitic impedance.

In various embodiments, the current-voltage measurement circuit 710 may include a power supply port 745, a current measurement port 746, a first voltage measurement port 747, a second voltage measurement port 748, an AC signal generator 741, an ammeter 742, and/or a voltmeter 743. The power supply port 745 and the current measurement port 746 may be electrically connected to the biometric sensor circuit 210 through the current-voltage path configuration circuit 750. The AC signal generator 741 may generate an electric signal and may apply the electric signal to the biometric sensor circuit 540 through the power supply port 745. The ammeter 742 may receive an electric signal through the current measurement port 746 from the biometric sensor circuit 540, measure the current of the received electric signal, and transfer the current measurement value to the processor 550. The first voltage measurement port 747 and the second voltage measurement port 748 may be electrically connected to the biometric sensor circuit 540 through the current-voltage path configuration circuit 750. The voltmeter 743 may measure the voltage between the first voltage measurement port 747 and the second voltage measurement port 748 and transfer the voltage measurement value to the processor 550.

In various embodiments, the current-voltage path configuration circuit 750 may electrically connect the ports 721, 722, 723 and 724 of the biometric sensor circuit 540 to the current-voltage measurement circuit 710 under the control of the processor 550.

In various embodiments, the processor 550 may control the current-voltage path configuration circuit 750 to construct a current path from one of the electrodes 541, 543, 545 and 547 to another. The processor 550 may control the current-voltage path configuration module 750 to construct a voltage path from one of the electrodes 541, 543, 545 or 547 to another. The processor 550 may control the current-voltage path configuration circuit 750 to construct a voltage path in a manner that a first one of two electrodes of the voltage path is identical to a first one of two electrodes of the current path, a second one of the two electrodes of the voltage path is different from a second one of the two electrodes of the current path, and the bio-impedance $Z_B$ is not included in the voltage path. For example, the processor 550 may construct a current-voltage path in the biometric sensor circuit 540 so that the first electrode 541 is included in the current path and the voltage path, and $Z_B$ is not included in the voltage path. The processor 550 may receive current/voltage measurement values from the ammeter 742 and the voltmeter 743 in a state where the biometric sensor circuit 540 is configured with the above current-voltage path. The processor 550 may measure the parasitic impedance of the first electrode 541 (e.g., parasitic impedance $Z_{P1}$ between the first electrode 541 and ground) on the current/voltage path by using the received current/voltage values. The processor 550 may measure parasitic impedances $Z_{P2}$, $Z_{P3}$ and $Z_{P4}$ for the remaining electrodes 543, 545 and 547 in the same manner as described above.

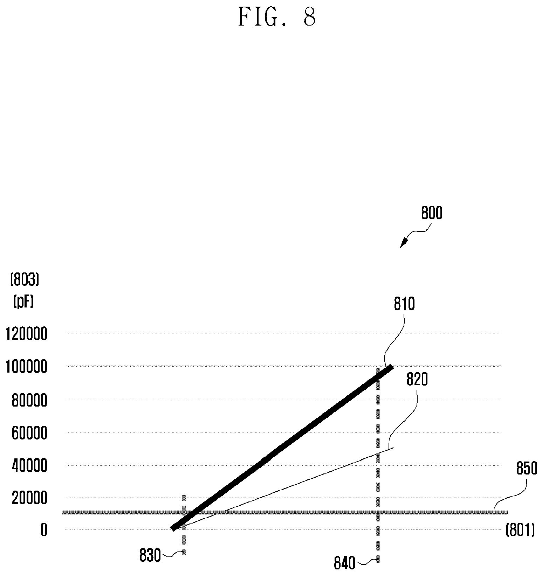

FIG. 8 is a diagram 800 for explaining a method for detecting moisture in the wearable electronic device 501 based on the parasitic impedance value of at least one electrode according to an embodiment of the disclosure.

FIG. 8 according to various embodiments may be a drawing for explaining operation 610 in FIG. 6 described above.

In FIG. 8, the x-axis may represent the state (801) of the wearable electronic device (e.g., wearable electronic device 501 of FIG. 5), and the y-axis may represent the parasitic impedance value (pF) (803) of an electrode according to the state of the wearable electronic device 501.

In an embodiment, the first graph 810 represents parasitic impedance values (e.g., $Z_{P3}$, $Z_{P4}$ in FIG. 7) of the third electrode (e.g., third electrode 545 in FIG. 5) and the fourth electrode (e.g., fourth electrode 547 in FIG. 5) in the state (830) where no moisture is detected in the wearable electronic device 501 and in the state (840) where moisture is detected. With reference to the first graph 810, the parasitic impedance value of the third electrode 545 and the fourth electrode 547 may be about 70 pF in the state (830) where no moisture is detected in the wearable electronic device 501, and the parasitic impedance value of the third electrode 545 and the fourth electrode 547 may be about 90,000 pF in the state (840) where moisture is detected in the wearable electronic device 501.

In an embodiment, the second graph 820 represents parasitic impedance values (e.g., $Z_{P1}$, $Z_{P2}$ in FIG. 7) of the first electrode (e.g., first electrode 541 in FIG. 5) and the second electrode (e.g., second electrode 543 in FIG. 5) in the state (830) where no moisture is detected in the wearable electronic device 501 and in the state (840) where moisture is detected. With reference to the second graph 820, the parasitic impedance value of the first electrode 541 and the second electrode 543 may be about 40 pF in the state (830) where no moisture is detected in the wearable electronic device 501, and the parasitic impedance value of the first electrode 541 and the second electrode 543 may be about 50,000 pF in the state (840) where moisture is detected in the wearable electronic device 501.

As seen in the first graph 810 and the second graph 820 according to various embodiments, the parasitic impedance value of an electrode in a state (830) where no moisture is detected may be different from the parasitic impedance value of the electrode in a state (840) where moisture is detected in the wearable electronic device 501.

In an embodiment, the wearable electronic device 501 may identify the parasitic impedance value of at least one electrode (e.g., first electrode 541, second electrode 543, third electrode 545, and/or fourth electrode 547), and may determine that moisture is detected in the wearable electronic device 501 if the parasitic impedance value of at least one electrode (e.g., first electrode 541, second electrode 543, third electrode 545, and/or fourth electrode 547) exceeds a specified value (850) (e.g., about 10000 pF).

The parasitic impedance values of an electrode based on the first graph 810 and the second graph 820 according to various embodiments are intended to facilitate description of the disclosure and are not limited to measured values.

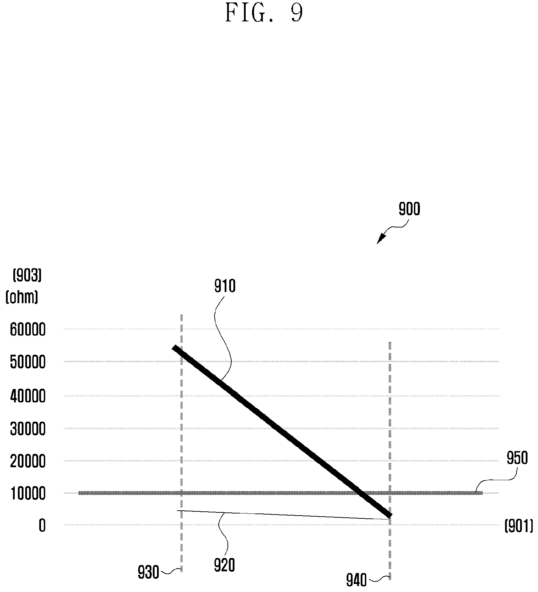

FIG. 9 is a diagram 900 for explaining a method for detecting moisture in the wearable electronic device 501 based on the resistance value of at least one electrode according to an embodiment of the disclosure.

FIG. 9 according to various embodiments may be a diagram for explaining operation 620 in FIG. 6 described above.

In FIG. 9, the x-axis may represent the state (901) of the wearable electronic device (e.g., wearable electronic device 501 in FIG. 5), and the y-axis may represent the resistance value (ohm) (903) of at least one electrode according to the state of the wearable electronic device 501.

In an embodiment, the third graph 910 represents the resistance value of the third electrode (e.g., third electrode 545 in FIG. 5) and the fourth electrode (e.g., fourth electrode 547 in FIG. 5) in a state (930) where no moisture is detected in the wearable electronic device 501 and in a state (940) where moisture is detected. With reference to the third graph 910, the resistance value of the third electrode 545 and the fourth electrode 547 may be about 50,000 ohm in the state (930) where no moisture is detected in the wearable electronic device 501, and the resistance value of the third electrode 545 and the fourth electrode 547 may be about 1,000 ohm in the state (940) where moisture is detected in the wearable electronic device 501.

In an embodiment, the fourth graph 920 represents the resistance value of the first electrode (e.g., first electrode 541 in FIG. 5) and the second electrode (e.g., second electrode 543 in FIG. 5) in the state (930) where no moisture is detected in the wearable electronic device 501 and in the state (940) where moisture is detected. With reference to the fourth graph 920, the resistance value of the first electrode 541 and the second electrode 543 may be about 5000 ohm in the state (930) where no moisture is detected in the wearable electronic device 501, and the resistance value of the first electrode 541 and the second electrode 543 may be about 1000 ohm in the state (940) where moisture is detected in the wearable electronic device 501.

As seen in the third graph 910 and the fourth graph 920 according to various embodiments, the resistance value of the electrode in the state (930) where no moisture is detected in the wearable electronic device 501 may be different from the resistance value of the electrode in the state (940) where moisture is detected. In particular, the difference between the resistance value of the first electrode 541 and the second electrode 543 in the state (930) where no moisture is detected in the wearable electronic device 501 and the resistance value of the first electrode 541 and the second electrode 543 in the state (940) where moisture is detected may be relatively small. The difference between the resistance value of the third electrode 545 and the fourth electrode 547 in the state (930) where moisture is not detected in the wearable electronic device 501 and the resistance value of the third electrode 545 and the fourth electrode 547 in the state (940) where moisture is detected may be greater than the difference between the resistance values in the corresponding states for the first electrode 541 and the second electrode 543.

In an embodiment, by utilizing the fact that the difference between the resistance value of the third electrode 545 and the fourth electrode 547 in the state (930) where moisture is not detected in the wearable electronic device 501 and the resistance value of the third electrode 545 and the fourth electrode 547 in the state (940) where moisture is detected is greater than the difference between the resistance values in the corresponding states for the first electrode 541 and the second electrode 543, the wearable electronic device 501 may identify the resistance value of at least one of the third electrode 545 and/or the fourth electrode 547 and determine that moisture is detected in the wearable electronic device 501 if the resistance value of at least one of the third electrode 545 and/or the fourth electrode 547 is less than or equal to a specified value (950) (e.g., approximately 10000 ohm).

In various embodiments, the wearable electronic device 501 may determine that moisture is detected in the wearable electronic device 501 if the parasitic impedance value of at least one electrode (e.g., first electrode 541, second electrode 543, third electrode 545, and/or fourth electrode 547) exceeds the specified value (850) (e.g., about 10000 pF) according to the embodiment of FIG. 8 described above, and then it may additionally perform the embodiment of FIG. 9 described above, thereby more accurately determining that moisture is detected in the wearable electronic device 501.

The resistance values of the electrodes in the first graph 910 and the second graph 920 according to various embodiments are intended to facilitate explanation of the disclosure and are not limited to measured values.

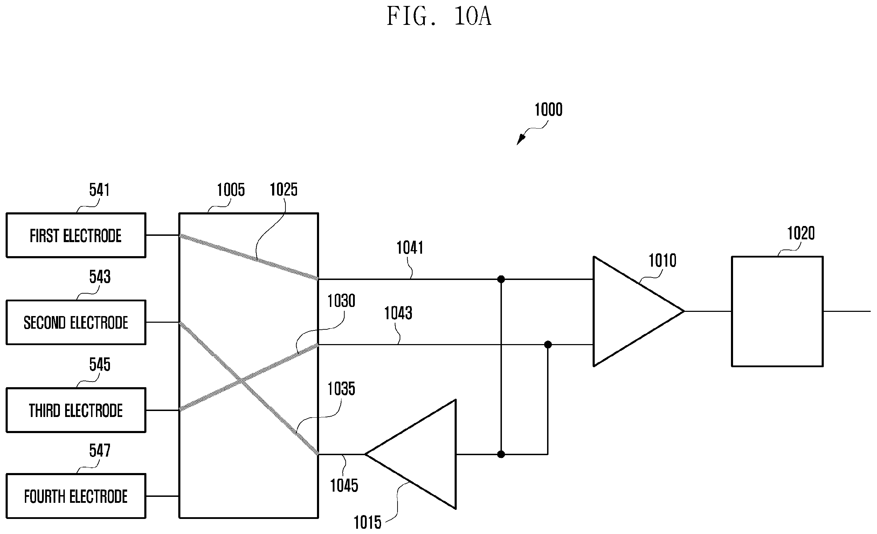

FIG. 10A is a diagram 1000 illustrating electrical connections between a plurality of electrodes when the wearable electronic device 501 obtains biometric information in a state of being worn according to an embodiment of the disclosure.

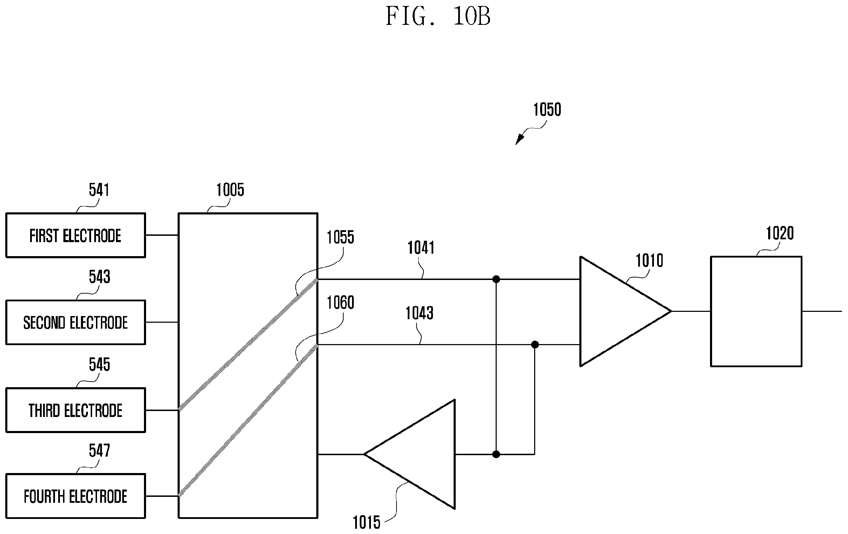

FIG. 10B is a diagram 1050 illustrating electrical connections between a plurality of electrodes when the wearable electronic device 501 does not obtain biometric information in a state of being worn according to an embodiment of the disclosure.

FIG. 10B according to various embodiments may be a diagram for explaining operation 630 in FIG. 6 described above.

With reference to FIG. 10A, the biometric sensor circuit (e.g., biometric sensor circuit 540 in FIG. 5) of the wearable electronic device (e.g., wearable electronic device 501 in FIG. 5) may include an electrocardiogram (ECG) sensor. In an embodiment, the ECG sensor may measure the electrical activity of the heart, e.g., electrocardiogram, by using a plurality of electrodes (e.g., first electrode 541, second electrode 543, third electrode 545, and/or fourth electrode 547 in FIG. 5).

For example, the first electrode 541 and the second electrode 543 may be disposed on the rear surface (e.g., rear surface 210B in FIG. 3) of the wearable electronic device 501, and the third electrode 545 and the fourth electrode 547 may be disposed on the side surface (e.g., side surface 210C in FIG. 3) of the wearable electronic device 501.

In an embodiment, the processor (e.g., processor 550 in FIG. 5) of the wearable electronic device 501 may measure the electrocardiogram by using at least three electrodes among the plurality of electrodes. For example, the electrocardiogram may be measured by using the first electrode 541 and the second electrode 543 disposed on the rear surface 210B of the wearable electronic device 501 and the third electrode 545 disposed on the side surface 210C of the wearable electronic device 501.

For example, the processor 550 may control the switching circuit 1005 to electrically connect the first electrode 541 to a first amplifier 1010 (e.g., instrumentation amplifier (INA)) through a first path 1041 (1025). The processor 550 may control the switching circuit 1005 to electrically connect the third electrode 545 to the first amplifier 1010 (e.g., INA) through a second path 1043 (1030). The processor 550 may control the switching circuit 1005 to electrically connect the second electrode 543 to a second amplifier 1015 (e.g., right-leg drive (RLD) amplifier) through a third path 1045 (1035). The first amplifier 1010 may be electrically connected to a converter 1020 (e.g., analog-to-digital converter (ADC)).

In an embodiment, although not shown, the converter 1020 (e.g., analog-to-digital converter (ADC)) may be electrically connected to the processor 550.

In an embodiment, the first amplifier 1010 electrically connected to the first electrode 541 and the third electrode 545 may generate a biometric signal by amplifying a signal input from the first electrode 541 and the third electrode 545 and reducing (or, removing) noise components. In an embodiment, the second amplifier 1015 electrically connected to the second electrode 543 may receive signals input from the first electrode 541 and the third electrode 545 in parallel and feed back (or, output) the signals to the second electrode 543. The feedback signal may be applied to the user's body through the second electrode 543. The second amplifier 1015 may aim to reduce (or, remove) noise components (e.g., power noise components (e.g., about 50 to 60 Hz)) by feeding them back to the user's body.

As described above, the processor 550 may measure the electrocardiogram by using the first electrode 541 and the third electrode 545, and feed back a feedback signal to the user's body through the second electrode 543 to reduce (or, remove) noise components, thereby improving electrocardiogram measurement performance.

In FIG. 10A according to various embodiments, it is described that the electrocardiogram is measured by using the first electrode 541, the second electrode 543, and the third electrode 545, but it is not limited thereto.

In an embodiment, when the processor 550 is not in a state of measuring biometric information by using a plurality of electrodes, it may monitor the impedance between the electrodes to determine whether a conductor is in contact with an electrode (e.g., lead-off).

For example, with reference to FIG. 10B, the processor 550 may control the switching circuit 1005 to release the electrical connection between the first electrode 541 and the first amplifier 1010. The processor 550 may control the switching circuit 1005 to release the electrical connection between the second electrode 543 and the second amplifier 1015. The processor 550 may control the switching circuit 1005 to electrically connect the third electrode 545 to the first amplifier 1010 through the first path 1041 (1055) instead of electrically connecting the third electrode 545 to the first amplifier 1010 through the second path 1043 (1030). The processor 550 may control the switching circuit 1005 to electrically connect the fourth electrode 547 to the first amplifier 1010 through the second path 1043 (1060).

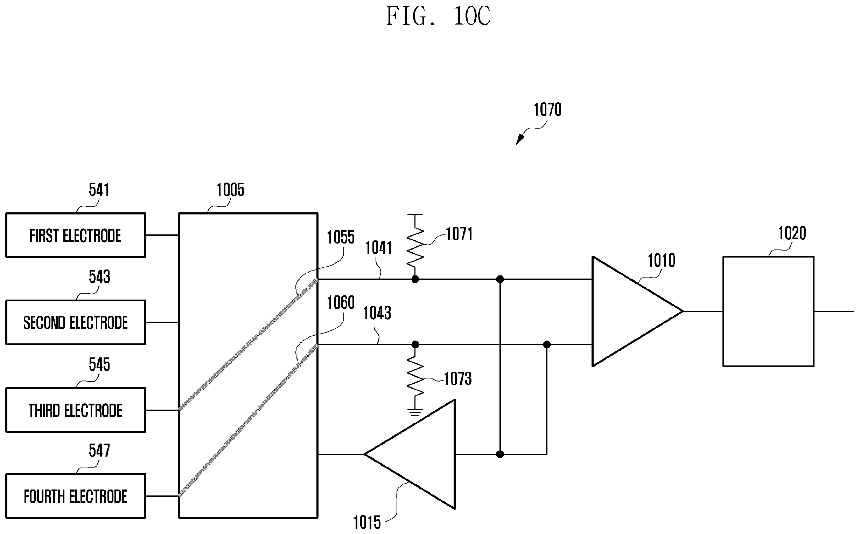

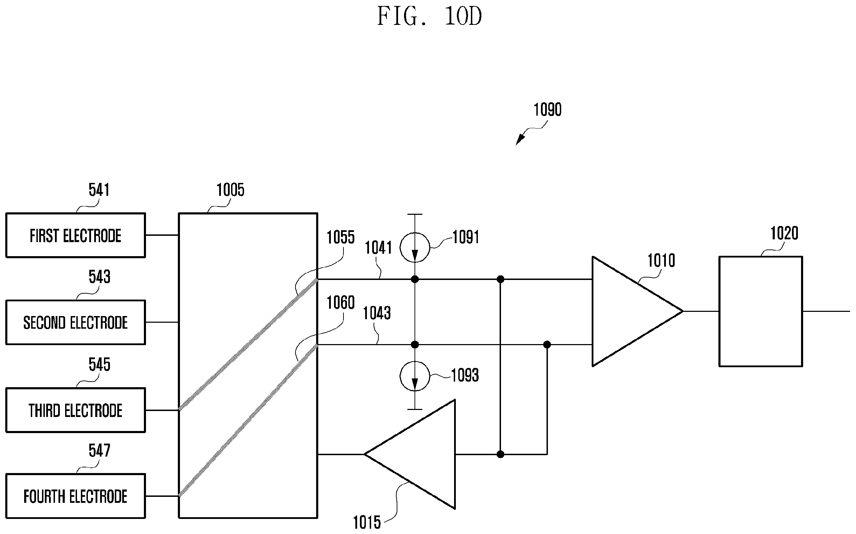

In an embodiment, in a state where the third electrode 545 and the fourth electrode 547 are electrically connected to the first amplifier 1010 (1055, 1060), the processor 550 may monitor the impedance of the third electrode 545 and the fourth electrode 547 to determine whether a conductor is in contact with the third electrode 545 and the fourth electrode 547. For example, the processor 550 may monitor the impedance of the third electrode 545 and the fourth electrode 547 by using the resistance or current of the third electrode 545 and the fourth electrode 547 to identify whether a conductor is in contact with the third electrode 545 and the fourth electrode 547. This will be described in FIGS. 10C and 10D below.

FIG. 10C is a diagram 1070 for explaining a method of monitoring the impedance of at least one electrode by using a resistor according to an embodiment of the disclosure.

With reference to FIG. 10C, when monitoring the impedance of the third electrode 545 and the fourth electrode 547 by using a resistor, the processor 550 may apply a pull-up resistance to the resistor 1071 connected to the third electrode 545 and apply a pull-down resistance to the resistor 1073 connected to the fourth electrode 547. In the above state, if a conductor comes into contact with the third electrode 545 and the fourth electrode 547 (e.g., in the case of contact), a short may occur at the third electrode 545 and the fourth electrode 547. Based on the occurrence of a short at the third electrode 545 and the fourth electrode 547, the processor 550 may identify the state of a conductor in contact with the third electrode 545 and/or the fourth electrode 547. Without being limited thereto, in a state where a pull-up resistance is applied to the resistor 1071 connected to the third electrode 545 and a pull-down resistance is applied to the resistor 1073 connected to the fourth electrode 547, the voltage value of the third electrode 545 and the fourth electrode 547 may change depending on the impedance of a conductor. The processor 550 may identify the state of the conductor in contact with the third electrode 545 and/or the fourth electrode 547 based on the changed voltage value of the third electrode 545 and the fourth electrode 547.

FIG. 10D is a diagram 1090 for explaining a method of monitoring the impedance of at least one electrode by using a current according to an embodiment of the disclosure.

In an embodiment, when monitoring the impedance of the third electrode 545 and the fourth electrode 547 by using a current, the processor 550 may apply a current to the ammeter 1091 connected to the third electrode 545 and the ammeter 1093 connected to the fourth electrode 547. In the above state, if a conductor comes into contact with the third electrode 545 and the fourth electrode 547, a short may occur at the third electrode 545 and the fourth electrode 547. Based on the occurrence of a short at the third electrode 545 and the fourth electrode 547, the processor 550 may identify the state of the conductor in contact with the third electrode 545 and/or the fourth electrode 547. Without being limited thereto, the impedance value of the conductor may change due to a current flowing in the ammeter 1091 connected to the third electrode 545 and the ammeter 1093 connected to the fourth electrode 547. The processor 550 may identify the state of a conductor in contact with the third electrode 545 and/or the fourth electrode 547 based on the changed impedance value of the third electrode 545 and/or the fourth electrode 547.

In various embodiments, the wearable electronic device 501 may determine that moisture is detected in the wearable electronic device 501 if the parasitic impedance value of at least one electrode (e.g., first electrode 541, second electrode 543, third electrode 545, and/or fourth electrode 547) exceeds the specified value (850) (e.g., about 10000 pF) according to the embodiment of FIGS. 8 and 9 described above, and then it may additionally perform the embodiment of FIGS. 10B to 10D described above, thereby more accurately determining that moisture is detected in the wearable electronic device 501.

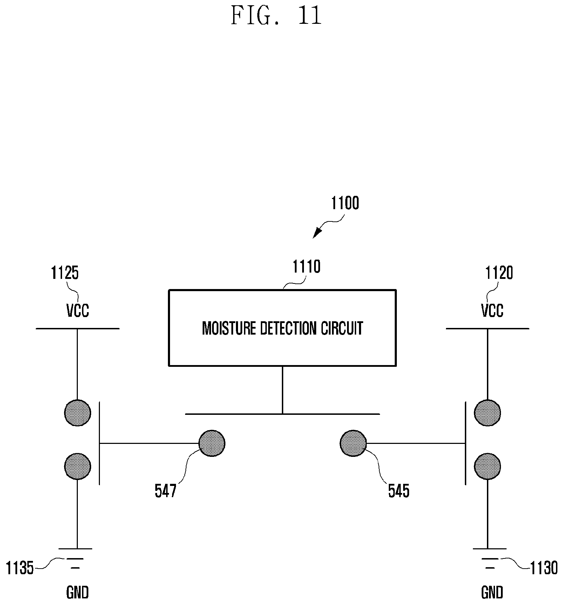

FIG. 11 is a diagram 1100 illustrating the moisture detection circuit 1110 of the wearable electronic device 501 according to an embodiment of the disclosure.

With reference to FIG. 11, the wearable electronic device (e.g., wearable electronic device 501 in FIG. 5) may include a moisture detection circuit 1110. For example, the moisture detection circuit 1110 may be disposed between the third electrode (e.g., third electrode 545 in FIG. 5) and the fourth electrode (e.g., fourth electrode 547 in FIG. 5) arranged on the side surface (e.g., side surface 210C in FIG. 3) of the wearable electronic device 501. Without being limited thereto, the moisture detection circuit 1110 may be disposed between the third electrode 545, the fourth electrode 547, and the housing (e.g., housing 210 in FIG. 2).

In an embodiment, the processor (e.g., processor 550 in FIG. 5) of the wearable electronic device 501 may detect moisture through the moisture detection circuit 1110. When moisture is detected through the moisture detection circuit 1110, the processor 550 may activate the biometric sensor circuit (e.g., biometric sensor circuit 540 in FIG. 5). For example, the biometric sensor circuit 540 may be activated by being powered through the VCC terminal 1120 or 1125 and the GND terminal 1130 or 1135 connected respectively to the third electrode 545 and the fourth electrode 547. Thereafter, the processor 550 may identify the parasitic impedance value of at least one electrode.

In various embodiments, instead of repeatedly identifying the parasitic impedance value of at least one of the first electrode 541, the second electrode 543, the third electrode 545, and/or the fourth electrode 547 at specified time points at operation 610 in FIG. 6 described above, in the embodiment of FIG. 11, when moisture is detected through the moisture detection circuit 1110, the biometric sensor circuit 540 may be activated to identify the parasitic impedance value of at least one electrode. Thereafter, the processor 550 may perform operations 620 to 640 in FIG. 6.

In FIG. 11 according to various embodiments, activating the biometric sensor circuit 540 to identify the parasitic impedance value of at least one electrode when moisture is detected through the moisture detection circuit 1110 may reduce current consumption in comparison to the case where the biometric sensor circuit 540 is repeatedly activated at specified time points to identify the parasitic impedance value of at least one electrode.

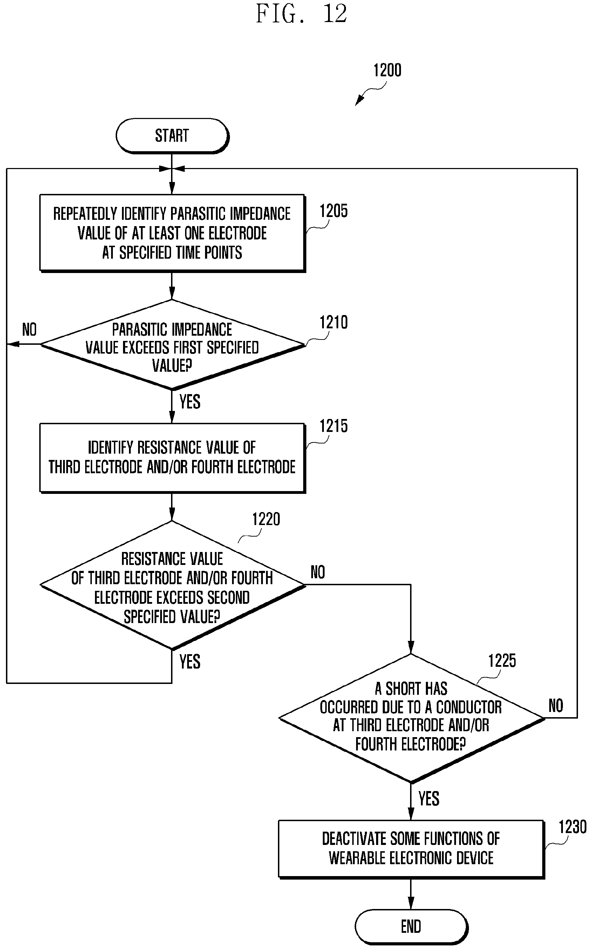

FIG. 12 is a flowchart 1200 for refining the operations of FIG. 6 according to an embodiment of the disclosure.

In the following embodiment, operations may be performed sequentially, but are not necessarily performed in sequence. For example, the order of operations may be changed, and at least two operations may be performed in parallel.

According to an embodiment, operations 1205 to 1230 may be understood to be executed by the processor (e.g., processor 550 in FIG. 5) of the wearable electronic device (e.g., wearable electronic device 501 in FIG. 5).

With reference to FIG. 12, at operation 1205, the processor 550 of the wearable electronic device 501 may repeatedly identify the parasitic impedance value of at least one electrode at designated time points.

In an embodiment, the wearable electronic device 501 may include a biometric sensor circuit (e.g., biometric sensor circuit 540 in FIG. 5). The biometric sensor circuit 540 may include a plurality of electrodes for obtaining biometric information of the user. The plurality of electrodes may include a first electrode (e.g., first electrode 541 in FIG. 5), a second electrode (e.g., second electrode 543 in FIG. 5), a third electrode (e.g., third electrode 545 in FIG. 5), and a fourth electrode (e.g., fourth electrode 547 in FIG. 5). For example, the first electrode 541 and the second electrode 543 may be disposed on the rear surface (e.g., rear surface 210B in FIG. 3) of the wearable electronic device 501 so as to come into contact with the user's wrist. The third electrode 545 and the fourth electrode 547 may be disposed on the side surface (e.g., side surface 210C in FIG. 3) of the wearable electronic device 501 so as to come into contact with a user's finger.

In an embodiment, the processor 550 may identify the parasitic impedance value of at least one electrode among the plurality of electrodes. In an embodiment, at operation 1210, the processor 550 may determine whether the parasitic impedance value of at least one electrode exceeds a specified first value (e.g., specified value (850) in FIG. 8 (e.g., about 10000 pF)).

In an embodiment, if the parasitic impedance value exceeds the specified first value (e.g., specified value (850) in FIG. 8 (e.g., about 10000 pF)) (e.g., YES at operation 1210), at operation 1215, the processor 550 may identify the resistance value of the third electrode 545 and/or the fourth electrode 547 disposed on the side surface (e.g., side surface 210C in FIG. 3). At operation 1220, the processor 550 may determine whether the resistance value of the third electrode 545 and/or the fourth electrode 547 exceeds a specified second value (e.g., specified value (950) in FIG. 9 (e.g., about 10000 ohm)).

In an embodiment, if the resistance value of the third electrode 545 and/or the fourth electrode 547 does not exceed a specified second value (e.g., specified value (950) in FIG. 9 (e.g., about 10000 ohm)) (e.g., NO at operation 1220), at operation 1225, the processor 550 may determine whether a short has occurred due to a conductor at the third electrode 545 and/or the fourth electrode 547. If it is determined that a short has occurred due to a conductor at the third electrode 545 and/or the fourth electrode 547 (e.g., YES at operation 1225), at operation 1230, the processor 550 may deactivate some functions of the wearable electronic device 501.

In an embodiment, if the parasitic impedance value of at least one electrode does not exceed the first specified value (e.g., specified value (850) in FIG. 8 (e.g., about 10,000 pF)) (e.g., NO at operation 1210), if the resistance value of the third electrode 545 and the fourth electrode 547 exceeds the second specified value (e.g., specified value (950) in FIG. 9 (e.g., about 10,000 ohm)) (e.g., YES at operation 1220), or if no short has occurred due to a conductor at the third electrode 545 and the fourth electrode 547 (e.g., NO at operation 1225), the processor 550 may determine that moisture is not detected in the wearable electronic device 501 and may proceed to operation 1205.

In FIG. 12 according to various embodiments, if the parasitic impedance value of at least one electrode exceeds the specified first value (e.g., specified value (850) in FIG. 8 (e.g., about 10,000 pF)), if the resistance value of the third electrode 545 and/or the fourth electrode 547 is less than or equal to the specified second value (e.g., specified value (950) in FIG. 9 (e.g., about 10,000 ohm)), and if a short has occurred due to a conductor at the third electrode 545 and/or the fourth electrode 547, the processor 550 may more accurately determine that moisture is detected in the wearable electronic device 501. According to the conditions described above, the processor 550 may more accurately identify a situation in which moisture is detected in the wearable electronic device 501 and deactivate some functions of the wearable electronic device 501, so it is possible to prevent battery discharge due to unnecessary functions that may be executed because of moisture, and also possible to prevent corrosion of electrodes included in the wearable electronic device 501 due to moisture.

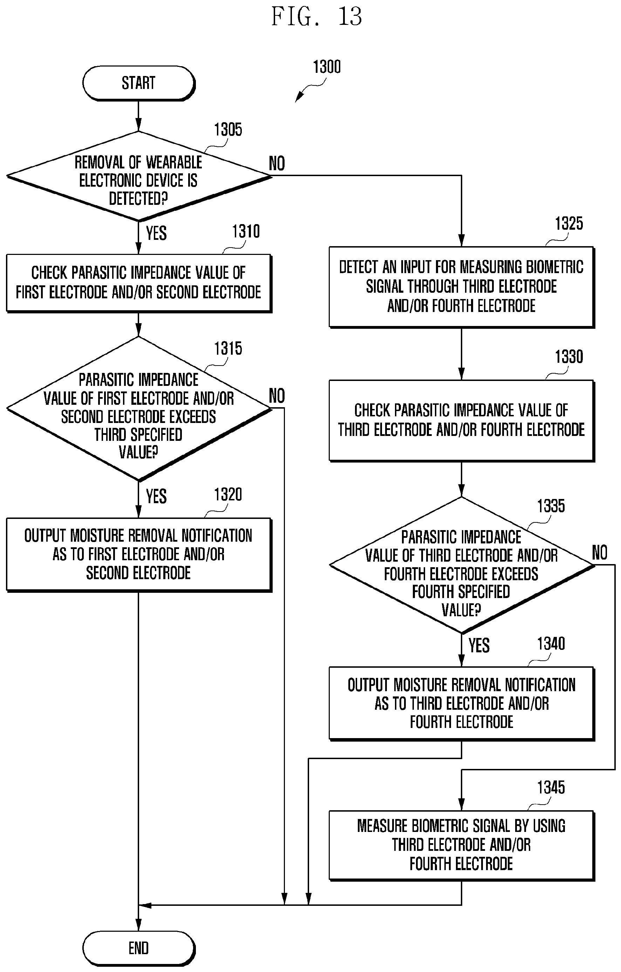

FIG. 13 is a flowchart 1300 for explaining a method of outputting a moisture removal notification in the wearable electronic device 501 according to an embodiment of the disclosure.

In the following embodiment, operations may be performed sequentially, but are not necessarily performed in sequence. For example, the order of operations may be changed, and at least two operations may be performed in parallel.

According to an embodiment, operations 1305 to 1345 may be understood to be performed by the processor (e.g., processor 550 in FIG. 5) of the wearable electronic device (e.g., wearable electronic device 501 in FIG. 5).

With reference to FIG. 13, at operation 1305, the processor 550 of the wearable electronic device 501 may determine whether removal of the wearable electronic device 501 has been detected. For example, the processor 550 may detect the wearing state of the wearable electronic device 501 through at least one sensor, such as an acceleration sensor, a geomagnetic sensor, a gyro sensor, and/or an IR sensor.

In an embodiment, upon detecting removal of the wearable electronic device 501 (e.g., YES at operation 1305), at operation 1310, the processor 550 may identify the parasitic impedance value (e.g., $Z_{P1}$ and/or $Z_{P2}$ in FIG. 7) of the first electrode (e.g., first electrode 541 in FIG. 5) and/or the second electrode (e.g., second electrode 543 in FIG. 5).

In an embodiment, at operation 1315, the processor 550 may determine whether the parasitic impedance value of the first electrode 541 and/or the second electrode 543 exceeds a specified third value (e.g., about 200 pF). If the parasitic impedance value of the first electrode 541 and/or the second electrode 543 exceeds the specified third value (e.g., about 200 pF) (e.g., YES at operation 1315), at operation 1320, the processor 550 may output a moisture removal notification as to the first electrode 541 and/or the second electrode 543.

For example, with reference to Table 1 below, when moisture is detected while the wearable electronic device 501 is not worn on the body, the parasitic impedance value of the first electrode 541 and/or the second electrode 543 may be about 250 pF, and when moisture is not detected while the wearable electronic device 501 is not worn on the body, the parasitic impedance value of the first electrode 541 and/or the second electrode 543 may be about 27 pF. The parasitic impedance values of the first electrode 541 and/or the second electrode 543 described in Table 1 below according to various embodiments are intended to facilitate explanation of the disclosure and are not limited to those given values.

[Table 1]

| Moisture detection state while wearable electronic device (501) is not worn on body | Parasitic impedance value of first electrode (541) | Parasitic impedance value of second electrode (543) |
|---|---|---|
| Moisture is detected while wearable electronic device (501) is not worn on body | about 250 pF | about 250 pF |
| Moisture is not detected while wearable electronic device (501) is not worn on body | about 27 pF | about 27 pF |

In an embodiment, if the parasitic impedance value of the first electrode 541 and/or the second electrode 543 exceeds a specified third value (e.g., about 200 pF), the processor 550 may determine that moisture is detected in the first electrode 541 and/or the second electrode 543, and output a moisture removal notification. For example, the moisture removal notification may be output through the display (e.g., display 530 in FIG. 5), the audio module (e.g., audio module 170 in FIG. 1), and/or the haptic module (e.g., haptic module 179 in FIG. 1).

In an embodiment, upon not detecting removal of the wearable electronic device 501 (e.g., NO at operation 1305), at operation 1325, the processor 550 may detect an input for measuring a biometric signal through the third electrode (e.g., third electrode 545 in FIG. 5) and/or the fourth electrode (e.g., fourth electrode 547 in FIG. 5). For example, an input for measuring a biometric signal through the third electrode 545 and/or the fourth electrode 547 may be an input by contacting a user's finger (e.g., a finger of the hand not wearing the wearable electronic device 501) for measuring a biometric signal.

In an embodiment, at operation 1330, the processor 550 may identify the parasitic impedance value (e.g., $Z_{P3}$ and/or $Z_{P4}$ in FIG. 7) of the third electrode 545 and/or the fourth electrode 547, and, at operation 1335, the processor 550 may determine whether the parasitic impedance value of the third electrode 545 and/or the fourth electrode 547 exceeds a specified fourth value (e.g., about 200 pF).

For example, with reference to Table 2 below, when moisture is not detected while the wearable electronic device 501 is worn on the body, the parasitic impedance value of the third electrode 545 may be about 43 pF, and the parasitic impedance value of the fourth electrode 547 may be about 45 pF. When moisture is detected while the wearable electronic device 501 is worn on the body, the parasitic impedance value of the third electrode 545 may be about 249 pF, and the parasitic impedance value of the fourth electrode 547 may be about 247 pF. The parasitic impedance values of the third electrode 545 and/or the fourth electrode 547 described in Table 2 below according to various embodiments are intended to facilitate explanation of the disclosure and are not limited to those given values.

[Table 2]

| Moisture detection state while wearable electronic device (501) is worn on body | Parasitic impedance value of third electrode (545) | Parasitic impedance value of fourth electrode (547) |
|---|---|---|
| Moisture is not detected while wearable electronic device (501) is worn on body | about 43 pF | about 45 pF |
| Moisture is detected while wearable electronic device (501) is worn on body | about 249 pF | about 247 pF |

In an embodiment, if the parasitic impedance value of the third electrode 545 and/or the fourth electrode 547 exceeds the specified fourth value (e.g., about 200 pF) (e.g., YES at operation 1335), at operation 1340, the processor 550 may output a moisture removal notification as to the third electrode 545 and/or the fourth electrode 547. For example, the moisture removal notification may be output through the display (e.g., display 530 in FIG. 5), the audio module (e.g., audio module 170 in FIG. 1), and/or the haptic module (e.g., haptic module 179 in FIG. 1).

In an embodiment, if the parasitic impedance value of the third electrode 545 and/or the fourth electrode 547 does not exceed the specified value (e.g., NO at operation 1335), in operation 1345, the processor 550 may measure a biometric signal by using the third electrode 545 and/or the fourth electrode 547.

In an embodiment, when an input for measuring a biometric signal through the third electrode 545 and/or the fourth electrode 547 is detected, above-described operations 1330 and 1335 are performed to provide a moisture removal notification when moisture is detected in the third electrode 545 and/or the fourth electrode 547, so that the user may attempt biometric measurement after removing moisture detected in the electrode. Hence, when attempting biometric measurement in a state where moisture is detected, it is possible to prevent corrosion of the electrode due to moisture.

In FIG. 13 according to various embodiments, operation 1305 for determining whether removal of the wearable electronic device 501 has been detected may be omitted.

For example, regardless of whether the wearable electronic device 501 is worn, it is possible to determine whether moisture is detected at the first electrode 541, the second electrode 543, the third electrode 545, and/or the fourth electrode 547 based on the parasitic impedance values of the first electrode 541, the second electrode 543, the third electrode 545, and/or the fourth electrode 547.

For example, even if removal of the wearable electronic device 501 is not detected, the processor 550 may determine whether moisture is detected at the first electrode 541 and/or the second electrode 543 based on the parasitic impedance values of the first electrode 541 and/or the second electrode 543.

In an embodiment, with reference to Table 3 below, when moisture is detected while the wearable electronic device 501 is worn on the body, the parasitic impedance value of the first electrode 541 and/or the second electrode 543 may be about 250 pF, and when moisture is not detected while the wearable electronic device 501 is worn on the body, the parasitic impedance value of the first electrode 541 and/or the second electrode 543 may be about 27 pF. The parasitic impedance values of the first electrode 541 and/or the second electrode 543 described in Table 3 below according to various embodiments are intended to facilitate explanation of the disclosure and are not limited to those given values.

[Table 3]

| Moisture detection state while wearable electronic device (501) is worn on body | Parasitic impedance value of first electrode (541) | Parasitic impedance value of second electrode (543) |
|---|---|---|
| Moisture is detected while wearable electronic device (501) is worn on body | about 250 pF | about 250 pF |
| Moisture is not detected while wearable electronic device (501) is worn on body | about 27 pF | about 27 pF |

In an embodiment, if the parasitic impedance value of the first electrode 541 and/or the second electrode 543 exceeds the specified third value (e.g., about 200 pF) while the wearable electronic device 501 is worn on the body, the processor 550 may determine that moisture is detected in the first electrode 541 and/or the second electrode 543, and output a moisture removal notification.

As another example, the processor 550 may determine whether moisture is detected at the third electrode 545 and/or the fourth electrode 547 based on the parasitic impedance values of the third electrode 545 and/or the fourth electrode 547 even when removal of the wearable electronic device 501 is detected.

In an embodiment, with reference to Table 4 below, when moisture is not detected while the wearable electronic device 501 is not worn on the body, the parasitic impedance value of the third electrode 545 may be about 43 pF, and the parasitic impedance value of the fourth electrode 547 may be about 45 pF. When moisture is detected while the wearable electronic device 501 is not worn on the body, the parasitic impedance value of the third electrode 545 may be about 249 pF, and the parasitic impedance value of the fourth electrode 547 may be about 247 pF. The parasitic impedance values of the third electrode 545 and/or the fourth electrode 547 described in Table 4 below according to various embodiments are intended to facilitate explanation of the disclosure and are not limited to those given values.

[Table 4]

| Moisture detection state while wearable electronic device (501) is not worn on body | Parasitic impedance value of third electrode (545) | Parasitic impedance value of fourth electrode (547) |
|---|---|---|
| Moisture is not detected while wearable electronic device (501) is not worn on body | about 43 pF | about 45 pF |
| Moisture is detected while wearable electronic device (501) is not worn on body | about 249 pF | about 247 pF |

In an embodiment, if the parasitic impedance value of the third electrode 545 and/or the fourth electrode 547 exceeds the specified fourth value (e.g., about 200 pF) while the wearable electronic device 501 is worn on the body, the processor 550 may determine that moisture is detected in the third electrode 545 and/or the fourth electrode 547, and output a moisture removal notification.

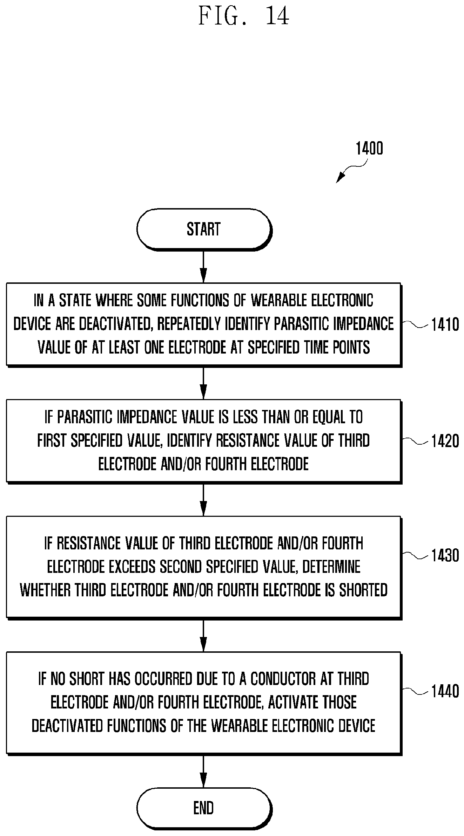

FIG. 14 is a flowchart 1400 for explaining a function control method of the wearable electronic device according to an embodiment of the disclosure.

In the following embodiment, operations may be performed sequentially, but are not necessarily performed in sequence. For example, the order of operations may be changed, and at least two operations may be performed in parallel.

According to an embodiment, operations 1410 to 1440 may be understood to be performed by the processor (e.g., processor 550 in FIG. 5) of the wearable electronic device (e.g., wearable electronic device 501 in FIG. 5).

FIG. 14 according to various embodiments may be additional operations for FIG. 6 or FIG. 12 described above.

With reference to FIG. 14, at operation 1410, while deactivating some functions of the wearable electronic device 501, the processor 550 of the wearable electronic device 501 may repeatedly identify the parasitic impedance value of at least one electrode at specified time points.

In an embodiment, some functions (e.g., touch functionality) of the wearable electronic device 501 may be deactivated based on determining that moisture is detected in the wearable electronic device 501 according to the embodiment of FIG. 6 or FIG. 12 described above.

In an embodiment, the biometric sensor circuit (e.g., biometric sensor circuit 540 in FIG. 5) may include a BIA sensor. In an embodiment, the BIA sensor may include a plurality of electrodes, for example, a first electrode (e.g., first electrode 541 in FIG. 5), a second electrode (e.g., second electrode 543 of FIG. 5), a third electrode (e.g., third electrode 545 in FIG. 5), and a fourth electrode (e.g., fourth electrode 547 in FIG. 5).

In an embodiment, the first electrode 541 and the second electrode 543 may be disposed on the rear surface (e.g., rear surface 210B in FIG. 3) of the wearable electronic device 501 so that they can come into contact with the user's wrist (e.g., the wrist of the hand wearing the wearable electronic device 501) when the wearable electronic device 501 is worn. The third electrode 545 and the fourth electrode 547 may be disposed on the side surface (e.g., side surface 210C in FIG. 3) of the wearable electronic device 501 so that they can come into contact with a user's finger (e.g., a finger of the hand not wearing the wearable electronic device 501).

In an embodiment, at operation 1420, if the parasitic impedance value of at least one electrode is less than or equal to a specified first value (e.g., specified value (850) in FIG. 8 (e.g., about 10000 pF)), the processor 550 may identify the resistance value of the third electrode 545 and/or the fourth electrode 547. For example, in a state where current is not likely to flow (e.g., no moisture is detected and/or being exposed to the air), the parasitic impedance value of at least one electrode may be less than or equal to about 100 pF, but without being limited thereto.

In an embodiment, the operation of determining whether the parasitic impedance value is less than or equal to the stored first value may be an operation of determining whether moisture is not detected in the wearable electronic device 501.

In an embodiment, at operation 1430, if the resistance value of the third electrode 545 and/or the fourth electrode 547 exceeds a specified second value (e.g., specified value (950) in FIG. 9 (e.g., about 10000 ohm)), the processor 550 may determine whether the third electrode 545 and/or the fourth electrode 547 is shorted.

In an embodiment, the operation of determining whether the resistance value of the third electrode 545 and/or the fourth electrode 547 exceeds the specified second value (e.g., specified value (950) in FIG. 9 (e.g., about 10000 ohm)) may be an operation of determining whether moisture is not detected in the wearable electronic device 501.

In an embodiment, at operation 1440, if no short has occurred due to a conductor at the third electrode 545 and/or the fourth electrode 547, the processor 550 may activate those deactivated functions of the wearable electronic device 501. For example, the processor 550 may monitor the impedance of the third electrode 545 and the fourth electrode 547 based on the resistance or current of the third electrode 545 and the fourth electrode 547 according to the aforementioned embodiments of FIGS. 10C and 10D and may determine whether a conductor is in contact with the third electrode 545 and the fourth electrode 547. In an embodiment, if no short has occurred due to a conductor not in contact with the third electrode 545 and the fourth electrode 547, the processor 550 may activate those deactivated functions of the wearable electronic device 501. For example, some functions of the wearable electronic device 501 may include, but not limited to, a touch functionality.

In operation 1410 according to various embodiments, it has been described that the parasitic impedance value of at least one electrode is repeatedly identified at specified time points, but it is not limited thereto. For example, according to the embodiment of FIG. 11 described above, the wearable electronic device 501 may include a moisture detection circuit 1110. When moisture is detected through the moisture detection circuit 1110, the processor 550 may activate the biometric sensor circuit 540 to identify the parasitic impedance value of at least one electrode.

In FIG. 14 according to various embodiments, if the parasitic impedance value of at least one electrode is less than or equal to the first specified value (e.g., specified value (850) in FIG. 8 (e.g., about 10000 pF)), if the resistance value of the third electrode 545 and/or the fourth electrode 547 exceeds the second specified value (e.g., specified value (950) in FIG. 9 (e.g., about 10000 ohm)), and if no short has occurred due to a conductor at the third electrode 545 and/or the fourth electrode 547, the processor 550 may accurately determine that no moisture is detected in the wearable electronic device 501.

In FIG. 14 according to various embodiments, it has been described that if the parasitic impedance value of at least one electrode is less than or equal to the first specified value, if the resistance value of the third electrode 545 and/or the fourth electrode 547 exceeds the second specified value, and if no short has occurred due to a conductor at the third electrode 545 and/or the fourth electrode 547, it is determined that moisture is not detected in the wearable electronic device 501, but without being limited thereto. For example, the processor 550 may determine that moisture is not detected in the wearable electronic device 501 based on at least two conditions (e.g., a condition in which the parasitic impedance value of at least one electrode is less than or equal to the first specified value and no short is caused by a conductor at the third electrode 545 and/or the fourth electrode 547, and a condition in which the resistance value of the third electrode 545 and/or the fourth electrode 547 exceeds the second specified value and no short is caused by a conductor at the third electrode 545 and/or the fourth electrode 547).

A function control method of the wearable electronic device 101, 200 or 501 according to various embodiments may include an operation of repeatedly identifying the parasitic impedance value of at least one electrode among a first electrode 271 or 541, a second electrode 272 or 543, a third electrode 273 or 545, and a fourth electrode 274 or 547 at specified time points. In an embodiment, the function control method of the wearable electronic device 101, 200 or 501 may include an operation of identifying the resistance value of at least one of the third electrode 273 or 545 or the fourth electrode 274 or 547 if the parasitic impedance value of at least one electrode exceeds a first specified value. In an embodiment, the function control method of the wearable electronic device 101, 200 or 501 may include an operation of identifying whether at least one of the third electrode 273 or 545 or the fourth electrode 274 or 547 is shorted if the resistance value of at least one of the third electrode 273 or 545 or the fourth electrode 274 or 547 is less than or equal to a second specified value. In an embodiment, the function control method of the wearable electronic device 101, 200 or 501 may include an operation of deactivating some functions of the wearable electronic device 101, 200 or 501 if a short occurs due to a conductor in at least one of the third electrode 273 or 545 or the fourth electrode 274 or 547.

In an embodiment, the function control method of the wearable electronic device 101, 200 or 501 may further include an operation of determining that moisture is detected in the wearable electronic device 101, 200 or 501 if a short has occurred due to a conductor in at least one of the third electrode 273 or 545 or the fourth electrode 274 or 547.

In an embodiment, the function control method of the wearable electronic device 101, 200 or 501 may further include an operation of connecting the first electrode 271 or 541 and the third electrode 273 or 545 to an AC current source. In an embodiment, the function control method of the wearable electronic device 101, 200 or 501 may further include an operation of connecting the second electrode 272 or 543 and the fourth electrode 274 or 547 to a voltage detector.

In an embodiment, the operation of identifying the parasitic impedance value of at least one electrode may include an operation of calculating an AC current generated from the AC current source and a voltage signal from the voltage detector to determine a parasitic impedance value according to parasitic components generated from at least one electrode.

In an embodiment, the operation of identifying whether at least one of the third electrode 273 or 545 or the fourth electrode 274 or 547 is shorted may include an operation of monitoring the impedance between the third electrode 273 or 545 and the fourth electrode 274 or 547 to determine whether a conductor is in contact with at least one of the third electrode 273 or 545 or the fourth electrode 274 or 547.

In an embodiment, the operation of determining whether a conductor is in contact with at least one of the third electrode 273 or 545 or the fourth electrode 274 or 547 may include an operation of identifying whether a conductor is in contact with at least one of the third electrode 273 or 545 or the fourth electrode 274 or 547 by using the resistance or current of at least one of the third electrode 273 or 545 or the fourth electrode 274 or 547.

In an embodiment, the operation of deactivating some functions of the wearable electronic device 101, 200 or 501 may include an operation of deactivating some functions of the wearable electronic device 101, 200 or 501 if a short occurs due to a conductor coming into contact with at least one of the third electrode 273 or 545 or the fourth electrode 274 or 547 while applying resistance or current to at least one of the third electrode 273 or 545 or the fourth electrode 274 or 547.

In an embodiment, the function control method of the wearable electronic device 101, 200 or 501 may further include an operation of identifying whether removal of the wearable electronic device 101, 200 or 501 is detected. In an embodiment, the function control method of the wearable electronic device 101, 200 or 501 may further include an operation of identifying the parasitic impedance value of at least one of the first electrode 271 or 541 or the second electrode 272 or 543 upon detecting removal of the wearable electronic device 101, 200 or 501. In an embodiment, the function control method of the wearable electronic device 101, 200 or 501 may further include an operation of outputting a moisture removal notification as to at least one of the first electrode 271 or 541 or the second electrode 272 or 543 if the parasitic impedance value of at least one of the first electrode 271 or 541 or the second electrode 272 or 543 exceeds a third specified value.

In an embodiment, the function control method of the wearable electronic device 101, 200 or 501 may further include an operation of identifying whether an input for measuring a biometric signal through at least one of the third electrode 273 or 545 or the fourth electrode 274 or 547 is detected upon not detecting removal of the wearable electronic device 101, 200 or 501. In an embodiment, the function control method of the wearable electronic device 101, 200 or 501 may further include an operation of identifying the parasitic impedance value of at least one of the third electrode 273 or 545 or the fourth electrode 274 or 547 upon detecting an input for measuring a biometric signal through at least one of the third electrode 273 or 545 or the fourth electrode 274 or 547. In an embodiment, the function control method of the wearable electronic device 101, 200 or 501 may further include an operation of outputting a moisture removal notification as to at least one of the third electrode 273 or 545 or the fourth electrode 274 or 547 if the parasitic impedance value of at least one of the third electrode 273 or 545 or the fourth electrode 274 or 547 exceeds a fourth specified value.

In an embodiment, the operation of outputting a moisture removal notification as to at least one electrode may include an operation of outputting a moisture removal notification as to the at least one electrode through at least one of the display 530, the audio output circuit 170, or the haptic module 179.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively," as "coupled with," "coupled to," "connected with," or "connected to" another element (e.g., a second element), it means that the element may be coupled with the other element directly (e.g., through wires), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

According to various embodiments, operations performed by the module, the program, or another component may be carried out sequentially, in parallel, repeatedly, or heuristically, or one or more of the operations may be executed in a different order or omitted, or one or more other operations may be added.

## Claims

1.  A wearable electronic device (101, 200, 501) comprising:

    a housing (210) including a first surface (210A), a second surface (210B) opposite to the first surface (210A), and a side surface (210C) surrounding the first surface (210A) and the second surface (210B);
    a first electrode (271, 541) and a second electrode (272, 543) disposed on the second surface (210B);
    a third electrode (273, 545) and a fourth electrode (274, 547) disposed on the side surface (210C); and
    a processor (550) operably connected to the first electrode (271, 541), the second electrode (272, 543), the third electrode (273, 545), and the fourth electrode (274, 547),
wherein the processor (550) is configured to:

repeatedly identify a parasitic impedance value of at least one electrode among the first electrode (271, 541), the second electrode (272, 543), the third electrode (273, 545), and the fourth electrode (274, 547) at specified time points;
identify a resistance value of at least one of the third electrode (273, 545) or the fourth electrode (274, 547) in case that the parasitic impedance value of the at least one electrode exceeds a first specified value;
determine whether at least one of the third electrode (273, 545) or the fourth electrode (274, 547) is shorted in case that the resistance value of at least one of the third electrode (273, 545) or the fourth electrode (274, 547) is less than or equal to a second specified value; and
deactivate some functions of the wearable electronic device (101, 200, 501) in case that a short has occurred due to a conductor in at least one of the third electrode (273, 545) or the fourth electrode (274, 547).

2. The wearable electronic device (101, 200, 501) of claim 1, wherein the processor (550) is configured to determine that moisture is detected in the wearable electronic device (101, 200, 501) in case that the short has occurred due to a conductor in at least one of the third electrode (273, 545) or the fourth electrode (274, 547).

3. The wearable electronic device of claim 1 or 2,

wherein the first electrode (271, 541) and the third electrode (273, 545) are connected to an AC current source;
wherein the second electrode (272, 543) and the fourth electrode (274, 547) are connected to a voltage detector; and
wherein the processor (550) is configured to calculate an AC current generated from the AC current source and a voltage signal of the voltage detector to identify the parasitic impedance value according to parasitic components generated from the at least one electrode.

4. The wearable electronic device of any of claims 1 to 3, wherein the processor (550) is configured to determine whether a conductor is in contact with at least one of the third electrode (273, 545) or the fourth electrode (274, 547) by monitoring an impedance between the third electrode (273, 545) and the fourth electrode (274, 547).

5. The wearable electronic device of claim 4, wherein the processor (550) is configured to determine whether a conductor is in contact with at least one of the third electrode (273, 545) or the fourth electrode (274, 547) by utilizing a resistance or current of at least one of the third electrode (273, 545) or the fourth electrode (274, 547).

6. The wearable electronic device of claim 4 or 5, wherein the processor (550) is configured to deactivate some functions of the wearable electronic device (101, 200, 501) in case that the short has occurred due to the conductor coming into contact with at least one of the third electrode (273, 545) or the fourth electrode (274, 547) in a state of applying a resistance or current to at least one of the third electrode (273, 545) or the fourth electrode (274, 547).

7. The wearable electronic device of claim 1, wherein the processor (550) is configured to:

identify whether removal of the wearable electronic device (101, 200, 501) is detected;
identify a parasitic impedance value of at least one of the first electrode (271, 541) or the second electrode (272, 543) in response to detecting removal of the wearable electronic device (101, 200, 501); and
output a moisture removal notification as to at least one of the first electrode (271, 541) or the second electrode (272, 543) in case that the parasitic impedance value of at least one of the first electrode (271, 541) or the second electrode (272, 543) exceeds a third specified value.

8. The wearable electronic device of claim 7, wherein the processor (550) is configured to:

identify whether an input for measuring a biometric signal through at least one of the third electrode (273, 545) or the fourth electrode (274, 547) is detected in response to not detecting removal of the wearable electronic device (101, 200, 501);
identify the parasitic impedance value of at least one of the third electrode (273, 545) or the fourth electrode (274, 547) in case that an input for measuring a biometric signal through at least one of the third electrode (273, 545) or the fourth electrode (274, 547) is detected; and
output a moisture removal notification as to at least one of the third electrode (273, 545) or the fourth electrode (274, 547) in case that the parasitic impedance value of at least one of the third electrode (273, 545) or the fourth electrode (274, 547) exceeds a fourth specified value.

9. The wearable electronic device of claim 7 or 8, further comprising:

   a display (530),
   an audio output circuit (170), and
   a haptic module (179),
   wherein the processor (550) is configured to output a moisture removal notification as to the at least one electrode through at least one of the display (530), the audio output circuit (170), or the haptic module (179).

10. A function control method of a wearable electronic device (101, 200, 501), the method comprising:

    repeatedly identifying a parasitic impedance value of at least one electrode among a first electrode (271, 541), a second electrode (272, 543), a third electrode (273, 545), and a fourth electrode (274, 547) at specified time points;
    identifying a resistance value of at least one of the third electrode (273, 545) or the fourth electrode (274, 547) in case that the parasitic impedance value of the at least one electrode exceeds a first specified value;
    determining whether at least one of the third electrode (273, 545) or the fourth electrode (274, 547) is shorted in case that the resistance value of at least one of the third electrode (273, 545) or the fourth electrode (274, 547) is less than or equal to a second specified value; and
    deactivating some functions of the wearable electronic device (101, 200, 501) in case that a short has occurred due to a conductor in at least one of the third electrode (273, 545) or the fourth electrode (274, 547).

11. The function control method of claim 10, further comprising determining that moisture is detected in the wearable electronic device (101, 200, 501) in case that the short has occurred due to a conductor in at least one of the third electrode (273, 545) or the fourth electrode (274, 547).

12. The function control method of claim 10 or 11, further comprising:

    connecting the first electrode (271, 541) and the third electrode (273, 545) to an AC current source; and
    connecting the second electrode (272, 543) and the fourth electrode (274, 547) to a voltage detector,
    wherein identifying a parasitic impedance value of at least one electrode comprises identifying the parasitic impedance value according to parasitic components generated from the at least one electrode by calculating an AC current generated from the AC current source and a voltage signal of the voltage detector.

13. The function control method of any of claims 10 to 12, wherein:

    determining whether at least one of the third electrode (273, 545) or the fourth electrode (274, 547) is shorted comprises determining whether a conductor is in contact with at least one of the third electrode (273, 545) or the fourth electrode (274, 547) by utilizing a resistance or current of at least one of the third electrode (273, 545) or the fourth electrode (274, 547); and
    deactivating some functions of the wearable electronic device (101, 200, 501) comprises deactivating some functions of the wearable electronic device (101, 200, 501) in case that the short has occurred due to the conductor coming into contact with at least one of the third electrode (273, 545) or the fourth electrode (274, 547) in a state of applying the resistance or current to at least one of the third electrode (273, 545) or the fourth electrode (274, 547).

14. The function control method of claim 10, further comprising:

    identifying whether removal of the wearable electronic device (101, 200, 501) is detected;
    identifying a parasitic impedance value of at least one of the first electrode (271, 541) or the second electrode (272, 543) in response to detecting removal of the wearable electronic device (101, 200, 501); and
    outputting a moisture removal notification as to at least one of the first electrode (271, 541) or the second electrode (272, 543) in case that the parasitic impedance value of at least one of the first electrode (271, 541) or the second electrode (272, 543) exceeds a third specified value.

15. The function control method of claim 14, further comprising:

identifying whether an input for measuring a biometric signal through at least one of the third electrode (273, 545) or the fourth electrode (274, 547) is detected in response to not detecting removal of the wearable electronic device (101, 200, 501);

identifying the parasitic impedance value of at least one of the third electrode (273, 545) or the fourth electrode (274, 547) in case that an input for measuring a biometric signal through at least one of the third electrode (273, 545) or the fourth electrode (274, 547) is detected; and

outputting a moisture removal notification as to at least one of the third electrode (273, 545) or the fourth electrode (274, 547) in case that the parasitic impedance value of at least one of the third electrode (273, 545) or the fourth electrode (274, 547) exceeds a fourth specified value.

# FIG. 1

ELECTRONIC DEVICE 101

100

INPUT MODULE 150

SOUND OUTPUT MODULE 155

DISPLAY MODULE 160

MEMORY 130
VOLATILE MEMORY 132
NON-VOLATILE MEMORY 134
INTERNAL MEMORY 136
EXTERNAL MEMORY 138

PROGRAM 140
APPLICATIONS 146
MIDDLEWARE 144
OPERATING SYSTEM 142

BATTERY 189

PROCESSOR 120
MAIN PROCESSOR 121
AUXILIARY PROCESSOR 123

POWER MANAGEMENT MODULE 188

COMMUNICATION MODULE 190
WIRELESS COMMUNICATION MODULE 192
WIRED COMMUNICATION MODULE 194

SUBSCRIBER IDENTIFICATION MODULE 196

ANTENNA MODULE 197

AUDIO MODULE 170

SENSOR MODULE 176

HAPTIC MODULE 179

CAMERA MODULE 180

INTERFACE 177

CONNECTION TERMINAL 178

SECOND NETWORK 199

ELECTRONIC DEVICE 104

FIRST NETWORK 198

ELECTRONIC DEVICE 102

SERVER 108

EP 4 574 043 A1

FIG. 2

# FIG. 3

FIG. 4

200

420

410

201

220

450

460

470

480

490

455

493

495

497

# FIG. 5

500

501

| 510 | 550 | 530 |
|---|---|---|

**WIRELESS COMMUNICATION CIRCUIT**

**PROCESSOR**

**DISPLAY**

520

**MEMORY**

540

**BIOMETRIC SENSOR CIRCUIT**

| FIRST ELECTRODE | 541 |
|---|---|
| SECOND ELECTRODE | 543 |
| THIRD ELECTRODE | 545 |
| FOURTH ELECTRODE | 547 |

FIG. 6

600

START

REPEATEDLY IDENTIFY PARASITIC IMPEDANCE VALUE OF AT LEAST ONE ELECTRODE AT SPECIFIED TIME POINTS ～610

IF PARASITIC IMPEDANCE VALUE EXCEEDS FIRST SPECIFIED VALUE, IDENTIFY RESISTANCE VALUE OF THIRD ELECTRODE AND/OR FOURTH ELECTRODE ～620

IF RESISTANCE VALUE OF THIRD ELECTRODE AND/OR FOURTH ELECTRODE IS LESS THAN OR EQUAL TO SECOND SPECIFIED VALUE, DETERMINE WHETHER THIRD ELECTRODE AND/OR FOURTH ELECTRODE ARE SHORTED ～630

IF A SHORT OCCURS DUE TO A CONDUCTOR AT THIRD ELECTRODE AND/OR FOURTH ELECTRODE, DEACTIVATE SOME FUNCTIONS OF WEARABLE ELECTRONIC DEVICE ～640

END

FIG. 7

EP 4 574 043 A1

FIG. 8

FIG. 9

# FIG. 10A

EP 4 574 043 A1

FIG. 10B

1050

1020

1010

1041

1043

1015

1005

1055

1060

541 FIRST ELECTRODE

543 SECOND ELECTRODE

545 THIRD ELECTRODE

547 FOURTH ELECTRODE

FIG. 10C

EP 4 574 043 A1

FIG. 10D

FIG. 11

FIG. 12

1200

START

REPEATEDLY IDENTIFY PARASITIC IMPEDANCE VALUE OF AT LEAST ONE ELECTRODE AT SPECIFIED TIME POINTS — 1205

PARASITIC IMPEDANCE VALUE EXCEEDS FIRST SPECIFIED VALUE? — 1210
NO
YES

IDENTIFY RESISTANCE VALUE OF THIRD ELECTRODE AND/OR FOURTH ELECTRODE — 1215

RESISTANCE VALUE OF THIRD ELECTRODE AND/OR FOURTH ELECTRODE EXCEEDS SECOND SPECIFIED VALUE? — 1220
NO
YES

A SHORT HAS OCCURRED DUE TO A CONDUCTOR AT THIRD ELECTRODE AND/OR FOURTH ELECTRODE? — 1225
NO
YES

DEACTIVATE SOME FUNCTIONS OF WEARABLE ELECTRONIC DEVICE — 1230

END

# FIG. 13

1300

START

1305
REMOVAL OF WEARABLE ELECTRONIC DEVICE IS DETECTED?

NO →

YES

1310
CHECK PARASITIC IMPEDANCE VALUE OF FIRST ELECTRODE AND/OR SECOND ELECTRODE

1315
PARASITIC IMPEDANCE VALUE OF FIRST ELECTRODE AND/OR SECOND ELECTRODE EXCEEDS THIRD SPECIFIED VALUE?

NO →

YES

1320
OUTPUT MOISTURE REMOVAL NOTIFICATION AS TO FIRST ELECTRODE AND/OR SECOND ELECTRODE

1325
DETECT AN INPUT FOR MEASURING BIOMETRIC SIGNAL THROUGH THIRD ELECTRODE AND/OR FOURTH ELECTRODE

1330
CHECK PARASITIC IMPEDANCE VALUE OF THIRD ELECTRODE AND/OR FOURTH ELECTRODE

1335
PARASITIC IMPEDANCE VALUE OF THIRD ELECTRODE AND/OR FOURTH ELECTRODE EXCEEDS FOURTH SPECIFIED VALUE?

NO →

YES

1340
OUTPUT MOISTURE REMOVAL NOTIFICATION AS TO THIRD ELECTRODE AND/OR FOURTH ELECTRODE

1345
MEASURE BIOMETRIC SIGNAL BY USING THIRD ELECTRODE AND/OR FOURTH ELECTRODE

END

# FIG. 14

1400

START

IN A STATE WHERE SOME FUNCTIONS OF WEARABLE ELECTRONIC DEVICE ARE DEACTIVATED, REPEATEDLY IDENTIFY PARASITIC IMPEDANCE VALUE OF AT LEAST ONE ELECTRODE AT SPECIFIED TIME POINTS — 1410

IF PARASITIC IMPEDANCE VALUE IS LESS THAN OR EQUAL TO FIRST SPECIFIED VALUE, IDENTIFY RESISTANCE VALUE OF THIRD ELECTRODE AND/OR FOURTH ELECTRODE — 1420

IF RESISTANCE VALUE OF THIRD ELECTRODE AND/OR FOURTH ELECTRODE EXCEEDS SECOND SPECIFIED VALUE, DETERMINE WHETHER THIRD ELECTRODE AND/OR FOURTH ELECTRODE IS SHORTED — 1430

IF NO SHORT HAS OCCURRED DUE TO A CONDUCTOR AT THIRD ELECTRODE AND/OR FOURTH ELECTRODE, ACTIVATE THOSE DEACTIVATED FUNCTIONS OF THE WEARABLE ELECTRONIC DEVICE — 1440

END

## INTERNATIONAL SEARCH REPORT

| International application No. |
| --- |
| **PCT/KR2023/011774** |

### A. CLASSIFICATION OF SUBJECT MATTER

**A61B 5/276**(2021.01)i; **A61B 5/00**(2006.01)i; **G06F 1/16**(2006.01)i; **G06F 1/3231**(2019.01)i; **G06F 11/30**(2006.01)i; **G06F 11/32**(2006.01)i

According to International Patent Classification (IPC) or to both national classification and IPC

### B. FIELDS SEARCHED

Minimum documentation searched (classification system followed by classification symbols)

A61B 5/276(2021.01); A61B 5/00(2006.01); A61B 5/053(2006.01); G06F 1/16(2006.01); H04B 1/3827(2015.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블 기기(wearable device), 전극(electrode), 임피던스(impedance), 단락 (short), 비활성화(deactivation)

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2022-0009884 A (SAMSUNG ELECTRONICS CO., LTD.) 25 January 2022 (2022-01-25)<br>See paragraphs [0019], [0046] and [0056]-[0057]; claims 1 and 21; and figures 1-7. | 1-15 |
| A | KR 10-2022-0082291 A (SAMSUNG ELECTRONICS CO., LTD.) 17 June 2022 (2022-06-17)<br>See paragraphs [0118]-[0119]; and figures 1-4 and 10. | 1-15 |
| A | KR 10-2017-0041501 A (LG ELECTRONICS INC.) 17 April 2017 (2017-04-17)<br>See paragraphs [0133]-[0190]; and figures 2-6. | 1-15 |
| A | US 2021-0204876 A1 (APPLE INC.) 08 July 2021 (2021-07-08)<br>See paragraphs [0004]-[0008]. | 1-15 |
| A | WO 2016-061381 A1 (ATLASENSE BIOMED LTD.) 21 April 2016 (2016-04-21)<br>See paragraphs [0005]-[0018]. | 1-15 |

☐ Further documents are listed in the continuation of Box C.   ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **20 November 2023** | **20 November 2023** |

| Name and mailing address of the ISA/KR | Authorized officer |
| --- | --- |
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/011774**

| Patent document cited in search report | | | Publication date (day/month/year) | Patent family member(s) | | | Publication date (day/month/year) |
|---|---|---|---|---|---|---|---|
| KR | 10-2022-0009884 | A | 25 January 2022 | CN | 116133583 | A | 16 May 2023 |
| | | | | EP | 4166075 | A1 | 19 April 2023 |
| | | | | US | 2023-0144358 | A1 | 11 May 2023 |
| | | | | WO | 2022-015081 | A1 | 20 January 2022 |
| KR | 10-2022-0082291 | A | 17 June 2022 | WO | 2022-124551 | A1 | 16 June 2022 |
| KR | 10-2017-0041501 | A | 17 April 2017 | KR | 10-2018-0033976 | A | 04 April 2018 |
| | | | | US | 10187164 | B2 | 22 January 2019 |
| | | | | US | 2018-0316442 | A1 | 01 November 2018 |
| | | | | WO | 2017-061722 | A1 | 13 April 2017 |
| US | 2021-0204876 | A1 | 08 July 2021 | CN | 111065984 | A | 24 April 2020 |
| | | | | CN | 209789844 | U | 17 December 2019 |
| | | | | CN | 213372029 | U | 08 June 2021 |
| | | | | EP | 3451117 | A1 | 06 March 2019 |
| | | | | EP | 3451117 | B1 | 23 August 2023 |
| | | | | KR | 10-2020-0027010 | A | 11 March 2020 |
| | | | | KR | 10-2022-0011803 | A | 28 January 2022 |
| | | | | KR | 10-2023-0087624 | A | 16 June 2023 |
| | | | | KR | 10-2352800 | B1 | 18 January 2022 |
| | | | | KR | 10-2543660 | B1 | 14 June 2023 |
| | | | | US | 10610157 | B2 | 07 April 2020 |
| | | | | US | 10987054 | B2 | 27 April 2021 |
| | | | | US | 11432766 | B2 | 06 September 2022 |
| | | | | US | 2019-0072912 | A1 | 07 March 2019 |
| | | | | US | 2019-0101870 | A1 | 04 April 2019 |
| | | | | US | 2020-0229761 | A1 | 23 July 2020 |
| | | | | US | 2023-0098960 | A1 | 30 March 2023 |
| | | | | US | 2023-0210461 | A1 | 06 July 2023 |
| | | | | WO | 2019-050778 | A1 | 14 March 2019 |
| WO | 2016-061381 | A1 | 21 April 2016 | US | 11076763 | B2 | 03 August 2021 |
| | | | | US | 2017-0238812 | A1 | 24 August 2017 |
| | | | | US | 2022-0192513 | A1 | 23 June 2022 |

Form PCT/ISA/210 (patent family annex) (July 2022)